# EUROPÄISCHE PATENTANMELDUNG

(11) **EP 4 275 681 A1**
(43) Veröffentlichungstag der Anmeldung: **15.11.2023**
(21) Anmeldenummer: 22172462.8
(22) Anmeldetag: 10.05.2022
(51) Int. Cl.: A61K 31/245, A61K 31/167, A61P 11/00, A61P 31/16, A61P 43/00

(54) **WIRKSTOFFE ZUR MEDIZINISCHEN VERWENDUNG**

(71) Anmelder: inflamed pharma GmbH, 07745 Jena (DE)
(72) Erfinder: KÖNIG, Sarah, 37359 Großbartloff (DE); SCHROEDER, Josefine, 07743 Jena (DE); EHRHARDT, Christina, 07743 Jena (DE)
(74) Vertreter: Schiweck Weinzierl Koch Patentanwälte Partnerschaft mbB

(57) **Zusammenfassung**

Der Gegenstand der vorliegenden Erfindung betrifft ein Amin (AM) gemäß der allgemeinen Formel (I); ein Kohlensäure-Addukt (KA) und eine pharmazeutische Zusammensetzung (PZ) zur Verwendung in der Behandlung von Infektionen mit *Aspergillus fumigatus* und von Superinfektionen mit Influenzaviren und *Aspergillus spp..*

## Beschreibung

### TECHNISCHES GEBIET

Der Gegenstand der vorliegenden Erfindung betrifft ein Amin (AM) gemäß der allgemeinen Formel (I); ein Kohlensäure-Addukt (KA) und eine pharmazeutische Zusammensetzung (PZ) zur Verwendung in der Behandlung von Infektionen mit *Aspergillus fumigatus* und von Superinfektionen mit Influenzaviren und *Aspergillus spp..*

### TECHNISCHER HINTERGRUND

Influenzaviren verursachen weiterhin schwere Atemwegserkrankungen, die zu erheblicher Morbidität und Mortalität beitragen. Saisonale Epidemien sind für 3-5 Millionen schwere Fälle und schätzungsweise für 300.000-500.000 Todesfälle jedes Jahr weltweit verantwortlich (1). Darüber hinaus bergen Influenza-A-Viren das Potenzial, Pandemien zu verursachen, die in der Vergangenheit mehrere Millionen Todesopfer gefordert haben, wie beispielsweise der Ausbruch der Spanischen Grippe 1918/19 belegt (2). Während sich die meisten Menschen von einer Infektion erholen, kommt es bei anderen zu Komplikationen wie einer Lungenentzündung, die entweder durch das Virus selbst oder zusätzliche Krankheitserreger, einschließlich Bakterien, verursacht wird, unter denen *Streptococcus pneumoniae, Staphylococcus aureus* und *Haemophilus influenzae* eine wichtige Rolle spielen (3). Bei Superinfektionen mit Influenzaviren und Bakterien sind die Gründe für schwere Verläufe gut untersucht und neuartige Behandlungsmöglichkeiten in den Fokus des Interesses gerückt. Bei Superinfektionen mit Influenzaviren und Pilzen ist dies jedoch nicht der Fall (4, 5).

Dennoch wurde deutlich, dass Infektionen durch Pilze wie *Aspergillus spp.,* insbesondere *Aspergillus fumigatus,* bei Influenza Virus infizierten Patienten vorkommen und eine erhöhte Morbidität und Mortalität hervorrufen (5-9). Ein häufiges Problem von Sekundärinfektionen mit Pilzen ist, dass sie oft zu spät erkannt werden (10, 11). Neben der erhöhten Pathogenität bei Superinfektionen mit Influenzaviren und *Aspergillus fumigatus* ist die begrenzte Verfügbarkeit potenter Antiinfektiva gegen die verschiedenen Erreger von großer Bedeutung (5, 6, 12). Die hohe Variabilität der Influenzaviren und das ständige Auftreten neuer Stämme, sowie die schnelle Resistenzentwicklung von Influenzaviren gegen die verfügbaren Medikamente (13-16) in Kombination mit der späten Erkennung von Pilzinfektionen (10, 11), sind die Hauptgründe für die schlechten Behandlungsmöglichkeiten. Bisher ist die Behandlung von Superinfektionen von Influenzaviren und Pilzen mit einem einzigen Wirkstoff noch nicht möglich.

Auch wenn im vergangenen Jahrhundert intensive Forschungen und enorme Fortschritte bei den Behandlungsmöglichkeiten stattgefunden haben, stellen Influenzaviren nach wie vor eine ernsthafte Bedrohung für die Bevölkerung dar. Saisonale Epidemien sind auf die kontinuierlichen Punktmutationen im Genom von Influenza-A- und -B-Viren (Antigendrift) zurückzuführen, die zu einer Veränderung der Proteinstrukturen führen. Darüber hinaus haben Influenza-A-Viren das Potenzial, Pandemieausbrüche zu verursachen (2, 20-22). Bei einer Infektion mit mehr als einem Virussubtyp pro Individuum kann der Austausch von Gensegmenten (Antigenverschiebung) zu Virussubtypen führen, die die Eigenschaften der Elternstämme in einer neuen Kombination enthalten (20, 21). Infolgedessen ist das Immunsystem infizierter Wirte naiv und nicht in der Lage die neuen Pathogene effizient zu bekämpfen. Im letzten Jahrhundert kam es zu mehreren Pandemien, unter denen die "Spanische Grippe" von 1918/19 die verheerendsten Folgen hatte (23). Der Influenzavirusstamm, der für den Pandemieausbruch von 2009 verantwortlich ist, zirkuliert noch heute in der Bevölkerung zusammen mit der H3N2-Linie von 1968 und zwei verschiedenen Linien des Influenza-B-Virus (20).

Während virale und bakterielle Superinfektionen in den letzten Jahren gut untersucht wurden, ist über virale und fungale Superinfektionen wenig bekannt. Bakterielle Superinfektionen treten in der Regel innerhalb der ersten sieben Tage nach Influenzavirusinfektion auf, welche zu noch fulminanteren Erkrankungen, einhergehend mit Lungenentzündung und erhöhter Mortalität, führen (24, 25). In einigen Fällen tritt die bakterielle Superinfektion jedoch erst auf, wenn die Virusinfektion kuriert zu sein scheint. Es wurde mehrfach beschrieben, dass die Influenzavirusinfektion den Weg für eine bakterielle Superinfektion ebnet. Infolge einer Influenzavirusinfektion wird die Reinigungsfunktion der Zilien zerstört, die Schleimhautschicht aufgelöst und somit zusätzliche Rezeptoren für Bakterien freigelegt. Zudem ist die Immunantwort fehlreguliert, was zu einer verminderten Abwehr und vermehrten Entzündungsprozessen führt (4, 26).

In den letzten Jahren wurde auch berichtet, dass Patienten mit schwerer Influenzavirusinfektion eine invasive pulmonale Aspergillose entwickelten, die durch eine Superinfektion von Influenzaviren und Pilzen wie *Aspergillus spp.,* insbesondere *Aspergillus fumigatus,* hervorgerufen wurde. Dies führte zu einer noch höheren Sterblichkeitsrate im Vergleich zu einer Einfachinfektion mit Influenzaviren (27, 28).

*Aspergillus spp.* sind filamentöse saprotrophe Pilze, die im Boden und in der Luft vorkommen (11, 29). Bei gesunden Menschen werden die inhalativen Konidien durch mukoziliäre Reinigung und frühe Mechanismen der Immunabwehr bekämpft (11). Bei immungeschwächten oder transplantierten Patienten besteht ein erhöhtes Risiko von Komplikationen und gesteigerter Todesrate (5, 28). Die Wahrscheinlichkeit zur Entstehung einer invasiven pulmonalen Aspergillose, die durch das Eindringen der Hyphen von *Aspergillus fumigatus* in das Lungengewebe gekennzeichnet ist, ist bei immungeschwächten Patienten weiter erhöht und mit einer gesteigerten Mortalität verbunden (10). Eine relativ neue und unerforschte klinische Einheit ist die Post-Influenza-Aspergilliose, welche schwer zu diagnostizieren ist. Kürzlich wurde deutlich, dass Influenza-A- und B-Virusinfektionen für die Entwicklung von Superinfektionen mit Pilzen bei immungeschwächten und nicht immungeschwächten Patienten verantwortlich sind (6, 30, 31). Es wird angenommen, dass, ähnlich wie bei bakteriellen Superinfektionen, der Verlust der Ziliarfunktion der Schleimhaut den Patienten für eine Superinfektion mit Pilzen prädisponiert und die Entwicklung einer invasiven Pilzinfektion fördert (31).

Der bisher effizienteste Weg zum Schutz vor jährlichen Influenzavirus-Epidemien ist die Impfung (1, 13). Obwohl jedes Jahr neue Impfstoffe hergestellt werden, ist die Impfquote gering, die Effizienz ist variabel und im Falle gänzlich neu auftretender Influenza-A-Virus-Subtypen sind angepasste Impfstoffe nicht rechtzeitig verfügbar. Für jede neue Virusvariante müssen neue Impfstoffe angepasst und produziert werden, was mindestens sechs Monaten dauert. Somit ist zwischen dem Auftreten eines Erregers und der Einführung des neuen Impfstoffs ein Zeitraum vorhanden, in dem die Bevölkerung ungeschützt ist. Dementsprechend müssen antivirale Alternativen zur Kontrolle der Infektion eingesetzt werden.

Für die antivirale Therapie wurden von der Europäischen Arzneimittel-Agentur (EMA) drei Klassen von Verbindungen zugelassen, die entweder auf das lonenkanalprotein (M2), die Neuraminidase oder die CAP-abhängige Endonuklease abzielen. Während Neuraminidase-Inhibitoren gegen Influenza-A- und -B-Viren wirksam sind, versagen M2-Inhibitoren gegen Influenza-B-Viren (13). Leider entwickeln Influenzaviren schnell Resistenzen gegen Therapeutika. Mehrere Berichte weisen auf resistente Varianten von Influenza-B-Viren gegen Oseltamivir, sowie Influenza-A-Viren des H5N1- und des pandemischen H1N1v-Typen hin. Das häufige Auftreten resistenter Varianten in klinischen Isolaten gegen Adamantane führte zu der Empfehlung, M2-Inhibitoren nicht zur Behandlung und Prophylaxe einzusetzen, bis die Anfälligkeit für diese Arzneimittel bei zirkulierenden Influenza-A-Viren wiederhergestellt ist (16). Ein weiterer Schwachpunkt der bestehenden antiviralen Therapieansätze ist, dass die antivirale Behandlung so schnell wie möglich nach Beginn der Symptome eingeleitet werden muss.

Neuartige antivirale Strategien umfassen (a) die Hemmung virusunterstützender Zellfunktionen, (b) die Förderung der antiviralen Abwehr oder (c) die Auflösung von Entzündungen (32).

Die vielversprechendsten antiviralen Strategien zur Bekämpfung der Influenzainfektionen basieren auf der Tatsache, dass Influenzaviren als intrazelluläre Erreger stark von der zellulären Signalmaschinerie abhängen. Influenzaviren besitzen die Fähigkeit, zelluläre Faktoren für ihre eigenen Zwecke zu manipulieren, um ihre Replikation und Verbreitung zu gewährleisten. Darüber hinaus können Influenzaviren der angeborenen Immunantwort ihrer Wirte entgegenwirken. Angesichts dieser Abhängigkeiten stellen zelluläre virusunterstützende Funktionen Ziele für antivirale Interventionen dar (32). In den letzten Jahren konnten verschiedene zelluläre Faktoren als geeignete Ziele für eine antivirale Intervention identifiziert werden (33), darunter die Raf/MEK/ERK-Mitogenkinase-Kaskade (32, 34-39), das IKK/NFκB-Modul (40-42) und der PI3K-Signalweg (33, 43-48). Der Angriff der meisten dieser Faktoren erwies sich als wirksam gegen Influenzavirusinfektion *in vitro,* aber auch in *in vivo* Mausmodellen. Erste klinische Untersuchungen von LASAG deuten auf dessen antivirale Wirkung gegen Influenzavirusinfektion in hospitalisierten Patienten hin (61). Das Targeting zellulärer statt viraler Faktoren reduziert die Wahrscheinlichkeit der Entwicklung von Resistenzen, da das Pathogen die fehlende zellulare Funktion schwerer ausgleichen kann.

Unter den Antimykotika sind Triazole die erste Wahl gegen Aspergillosen, aber auch Echinocandine und das Polyen Amphotericin B werden eingesetzt (29). Die jüngste Entwicklung von Resistenzen bei *Aspergillus spp.,* insbesondere bei *Aspergillus fumigatus-Varianten,* ist besorgniserregend. Weltweit nahm die Azolresistenz zu und in der Folge empfehlen neue Leitlinien eine Voriconazol-Eninocandin-Kombination oder Amphotericin B (5). In der Regel wird die vorbeugende Behandlung der Aspergillose nicht eingeleitet. Erst wenn eine Infektion mit Pilzen naheliegt bzw. nachgewiesen wurde, wird eine empirische antifungale Therapie eingeleitet. Dabei handelt es sich um eine Kombination aus immunmodulatorischer Behandlung und antimykotischer Therapie (29).

Für die Behandlung von viralen/fungalen Superinfektionen gibt es nur begrenzte Möglichkeiten. Vielmehr ist eine Kortikosteroidbehandlung, die häufig auf Intensivstationen eingesetzt wird, aufgrund einer längeren Virusausscheidung mit erhöhter Mortalität und verstärkter invasiver pulmonaler Aspergillose kontraindiziert (5). Da neuere Studien auf eine starke Interferenz von *Aspergillus fumigatus* und seinem Wirt während der invasiven Aspergillose hindeuten, stellen gezielte Fungi-unterstützende zelluläre Faktoren und pilzinduzierte schädliche Entzündungsprozesse alternative Angriffsmöglichkeiten dar (29, 49, 50).

Aufgrund der phylogenetischen Beziehung zwischen Pilzen und Menschen gibt es verschiedene Faktoren, die sehr konserviert sind (51). So könnten diese Homologe gleichzeitig von einem chemischen Inhibitor angegriffen werden, der in der Folge auf die Wirtszelle, die *Aspergillus spp.* bzw. pathogeninduzierten Zellfunktionen wirkt. Unter diesen wurden die Mitogen-aktivierten Proteinkinase (MAPK) Kaskaden identifiziert (49, 52-54), welche fungale Prozesse, wie Biofilmbildung (55), Stresstoleranz (52, 53), Virulenz (49), sowie Wirtszellfunktionen wie inflammatorische Prozesse (50) regulieren. Interessanterweise wurde kürzlich gezeigt, dass Antimykotika wie Itraconazol eine hemmende Wirkung auf die Influenzavirusreplikation (56, 57) und die SARS-CoV-2-Infektion (58) haben. Auch wenn die Effekte wahrscheinlich auf unterschiedlichen Wirkmechanismen der Verbindung während der Replikation der unterschiedlichen Viren zurückzuführen sind, wird im Falle einer Influenzavirusinfektion die Blockade des Cholesterinexports aus dem endolysosomalen Kompartiment und die dadurch reduzierte Replikation diskutiert (56).
Bemerkenswerterweise wurden MAPK auch als potenzielle Ziele zur Influenzavirus-Intervention identifiziert, die an der viralen Replikation und virusinduzierten Hyperinflammation beteiligt sind (59, 60).

Daher besteht weiterer Bedarf an neuen Therapeutika zur Behandlung von Influenzaviren, *Aspergillus spp.* und insbesondere von Superinfektion von Influenzaviren und *Aspergillus spp..*

### ZUSAMMENFASSUNG DER ERFINDUNG

Die Erfindung bezieht sich auf ein Amin (AM) gemäß der allgemeinen Formel (I) 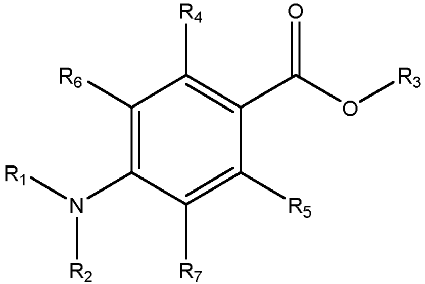 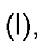 wobei in Formel (I)
R₁ ist H, (C₁₋₁₀)Alkyl, (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₂ ist H, (C₁₋₁₀ Alkyl), (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₃ ist -(CH₂)ₙNR₈R₉;
n ist 1 bis 5, vorzugsweise 1 bis 3, mehr bevorzugt 1 bis 2,
R₈ ist (C₁₋₁₀)Alkyl, vorzugsweise (C₁₋₂)Alkyl,
R₉ ist (C₁₋₁₀)Alkyl, vorzugsweise (C₁₋₂)Alkyl;
R₄ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀)Alkyl, vorzugsweise H, Halogen, (C₁₋₁₀)Alkyl oder -O(C₁₋₁₀) Alkyl, mehr bevorzugt H oder Halogen;
R₅ ist H, Halogen, (C₁₋₁₀)Alkyl, (C₂₋₁₀)Alkenyl, (C₅-C₁₄)Aryl, (C₅-C₁₀)Heteroaryl oder -O-(C₁₋₁₀) Alkyl, vorzugsweise H, Halogen, (C₁₋₁₀)Alkyl oder -O(C₁₋₁₀)Alkyl, mehr bevorzugt H oder Halogen;
R₆ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder -O(C₁₋₁₀)Alkyl, mehr bevorzugt H oder -O(C₁₋₁₀)Alkyl;
R₇ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder -O(C₁₋₁₀)Alkyl, mehr bevorzugt H oder -O-(C₁₋₁₀)Alkyl;
wobei das Amin gemäß Formel (I) optional auch in Form eines Salzes eingesetzt werden kann,
zur Verwendung in der Behandlung von Infektionen mit *Aspergillus fumigatus* und von Superinfektionen mit Influenzaviren und *Aspergillus spp..*

In einem weiteren Aspekt bezieht sich die Erfindung auf ein Kohlensäure-Addukt (KA) umfassend mindestens ein Strukturelement gemäß der allgemeinen Formel (II), (III) und/oder (IV) 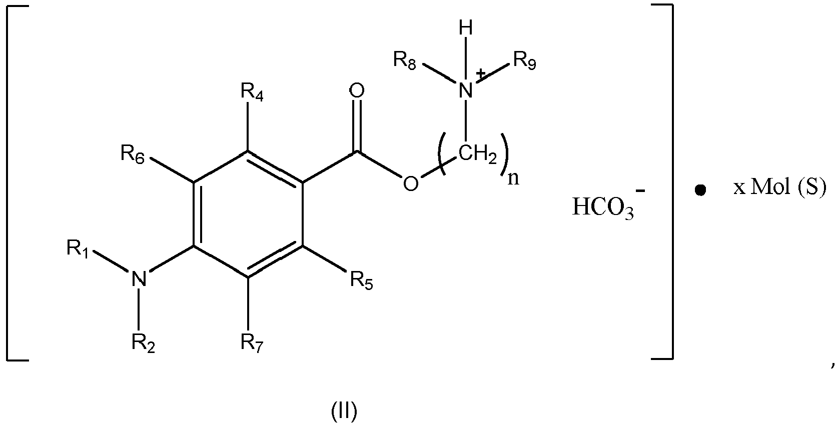 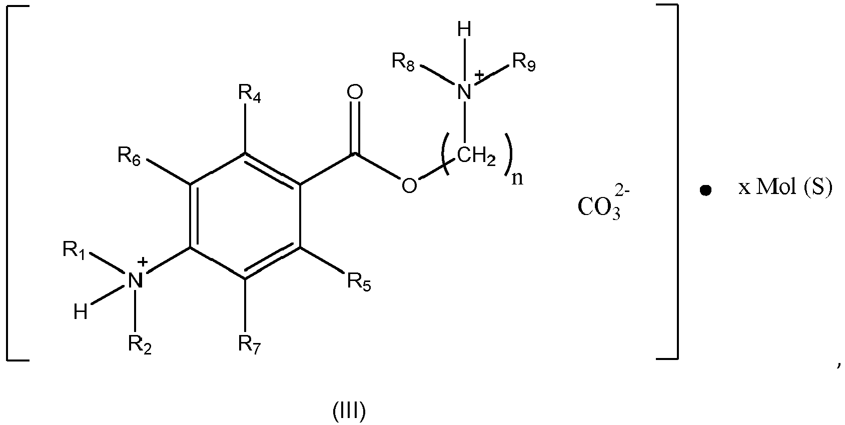 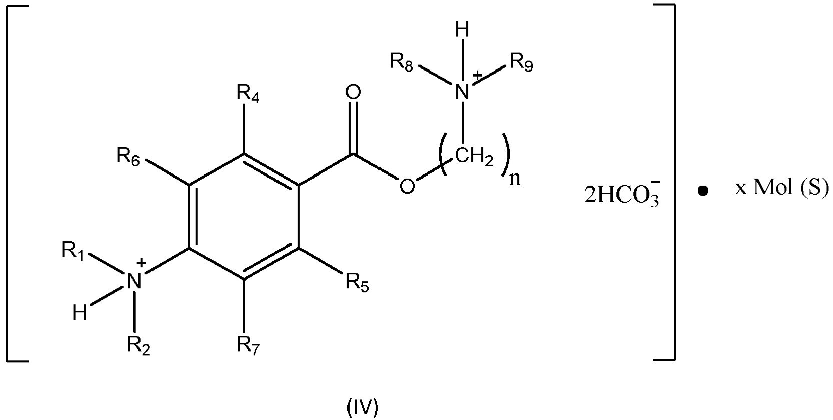 wobei in Formel (II), (III), und (IV)
R₁ ist H, (C₁₋₁₀)Alkyl, (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₂ ist H, (C₁₋₁₀ Alkyl), (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₃ ist -(CH₂)ₙNR₈R₉;
n ist 1 bis 5, vorzugsweise 1 bis 3, mehr bevorzugt 1 bis 2,
R₈ ist (C₁₋₁₀)Alkyl, vorzugsweise (C₁₋₂)Alkyl,
R₉ ist (C₁₋₁₀)Alkyl, vorzugsweise (C₁₋₂)Alkyl;
R₄ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀)Alkyl, vorzugsweise H, Halogen, (C₁₋₁₀)Alkyl oder -O(C₁₋₁₀) Alkyl, mehr bevorzugt H oder Halogen;
R₅ ist H, Halogen, (C₁₋₁₀)Alkyl, (C₂₋₁₀)Alkenyl, (C₅-C₁₄)Aryl, (C₅-C₁₀)Heteroaryl oder -O-(C₁₋₁₀) Alkyl, vorzugsweise H, Halogen, (C₁₋₁₀)Alkyl oder -O(C₁₋₁₀)Alkyl, mehr bevorzugt H oder Halogen;
R₆ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder -O(C₁₋₁₀)Alkyl, mehr bevorzugt H oder -O(C₁₋₁₀)Alkyl;
R₇ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder -O(C₁₋₁₀)Alkyl, mehr bevorzugt H oder -O(C₁₋₁₀)Alkyl;
x is 0,5 bis 30;
(S) ist ein Salz;
zur Verwendung in der Behandlung von Infektionen mit *Aspergillus fumigatus* und von Superinfektionen mit Influenzaviren und *Aspergillus spp..*

In einem weiteren Aspekt bezieht sich die Erfindung auf ein Kohlensäure-Addukt (KA) umfassend Kohlensäure, mindestens ein Amin (AM) gemäß der allgemeinen Formel (I) und mindestens ein Salz (S), 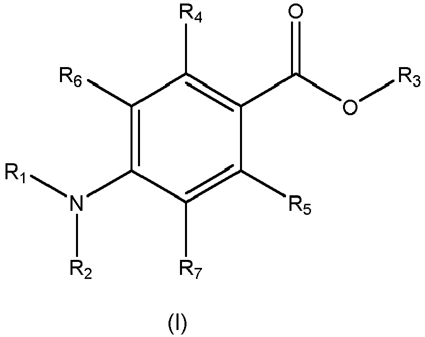 wobei in Formel (I)
R₁ ist H, (C₁₋₁₀)Alkyl, (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₂ ist H, (C₁₋₁₀ Alkyl), (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₃ ist -(CH₂)ₙNR₈R₉;
n ist 1 bis 5, vorzugsweise 1 bis 3, mehr bevorzugt 1 bis 2,
R₈ ist (C₁₋₁₀)Alkyl, vorzugsweise (C₁₋₂)Alkyl,
R₉ ist (C₁₋₁₀)Alkyl, vorzugsweise (C₁₋₂)Alkyl;
R₄ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀)Alkyl, vorzugsweise H, Halogen, (C₁₋₁₀)Alkyl oder -O(C₁₋₁₀) Alkyl, mehr bevorzugt H oder Halogen;
R₅ ist H, Halogen, (C₁₋₁₀)Alkyl, (C₂₋₁₀)Alkenyl, (C₅-C₁₄)Aryl, (C₅-C₁₀)Heteroaryl oder -O-(C₁₋₁₀) Alkyl, vorzugsweise H, Halogen, (C₁₋₁₀)Alkyl oder -O(C₁₋₁₀)Alkyl, mehr bevorzugt H oder Halogen;
R₆ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder -O(C₁₋₁₀)Alkyl, mehr bevorzugt H oder -O(C₁₋₁₀)Alkyl;
R₇ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder -O(C₁₋₁₀)Alkyl, mehr bevorzugt H oder -O(C₁₋₁₀)Alkyl;
wobei das mindestens eine Amin gemäß Formel (I) optional auch in Form eines Salzes eingesetzt werden kann;
herstellbar nach einem Verfahren umfassend die Schritte:
   a) Bereitstellung einer Lösung (A), die mindestens ein Lösungsmittel und in dem mindestens einem Lösungsmittel gelöstes CO₂ umfasst,
      optional b) Auflösen einer Base (BA), die nicht dem Amin (AM) entspricht, in der Lösung (A) unter Erhalt der Lösung (A1),
   c) Lösen des mindestens einen Amins (AM) in der Lösung (A) oder (A1), unter Erhalt der Lösung (B),
   d) Einfrieren der nach Abschluss des Schrittes c) erhaltenen Lösung,
   e) Lagerung der in Schritt d) eingefrorenen Lösung bei -100 bis 0 °C für nicht länger als 4 Tage;
   zur Verwendung in der Behandlung von Infektionen mit *Aspergillus fumigatus* und von Superinfektionen mit Influenzaviren und *Aspergillus spp..*

Die Erfindung bezieht sich weiterhin auf eine pharmazeutische Zusammensetzung umfassend das Amin, oder das Kohlensäure-Addukt zur Verwendung in der Behandlung von Infektionen mit *Aspergillus fumigatus* und von Superinfektionen mit Influenzaviren und *Aspergillus spp..*

Die Substanzen der hier zugrundeliegenden Erfindungsmeldung sind in der Lage, Influenzavirusinfektionen, sowie Influenzavirus-induzierte Zytokinexpression zu reduzieren und die Hyphenbildung von *Aspergillus fumigatus* zu blockieren. Somit bietet die vorliegende Erfindung einen neuen Ansatz zur anti-pathogenen Therapie beider Krankheitserreger mit einem einzigen Wirkstoff. Darüber hinaus ermöglicht die potenzielle Beeinflussung der Pathogen-induzierten zellulären Faktoren die Regulierung übermäßiger Immunantworten in Superinfektionsszenarien.

### KURZE BESCHREIBUNG DER FIGUREN

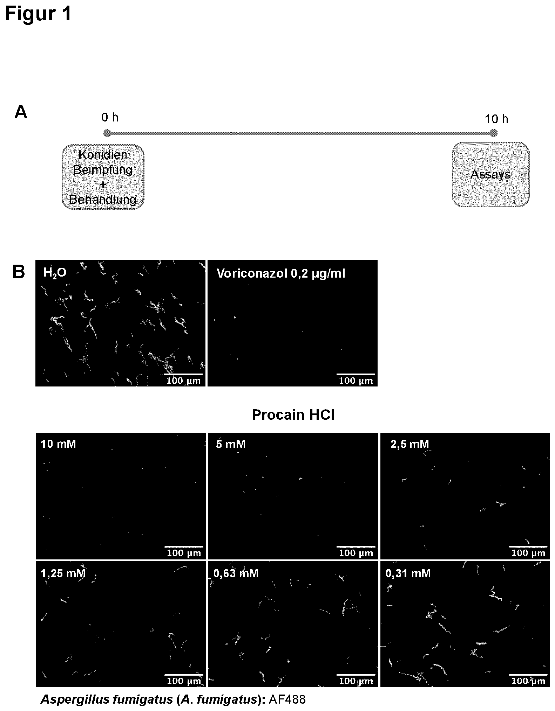

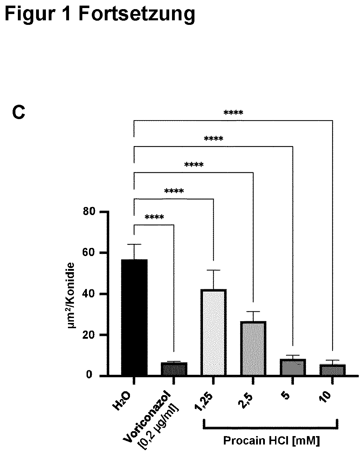

**Figur** 1: Procainhaltige Wirkstoffe (z.B. Procain-Hydrochlorid (HCl)) reduziert die Hyphenbildung von *Aspergillus fumigatus.*
   (A) Ablauf des Behandlungsverfahrens: Konidien eines GFP (*green-fluorescence protein*)-exprimierenden *Aspergillus fumigatus-Stammes* [5*10⁶ Konidien/Well] wurden auf Deckgläschen in 24 Well-Platten in 300 µl Medium DMEM/BA (0,2 % BSA, 1 mM MgCl₂ und 0,9 mM CaCl₂) ausgesät und jeweils supplementiert mit Lösungsmittel (H₂O), Procain HCl [0,31 (B) bzw. 1,25 (C) - 10 mM] oder Voriconazol [0,2 µg ml⁻¹] für 10 h bei 37 °C, 5 % CO₂ inkubiert. Anschließend wurden die Pilze fixiert (3,7 % Formaldehyd in PBS, 30 min, Raumtemperatur) und mit Hilfe von Fluoreszenz-Färbelösung (Agilent, Santa Clara, CA, USA) auf Objektträgern aufgebracht. Die Hyphengröße wurde mittels (B) Axio Observer.Z1 Mikroskop (Zeiss, Jena, Deutschland) und der Software Fiji V 1.52b (ImageJ) analysiert und (C) mit einem Partikelzähler berechnet. Die Daten stellen eine Repräsentation von vier Experimenten (B) oder den Mittelwert + SD von vier Experimenten mit jeweils zwei technischen Proben dar (C). Die statistische Signifikanz wurde mit der GraphPad Prism Software Version 9.2 unter Verwendung des Mehrfachvergleichstests von One-Way ANOVA berechnet. P-Werte sind durch Sternchen *p < 0,05 gekennzeichnet.

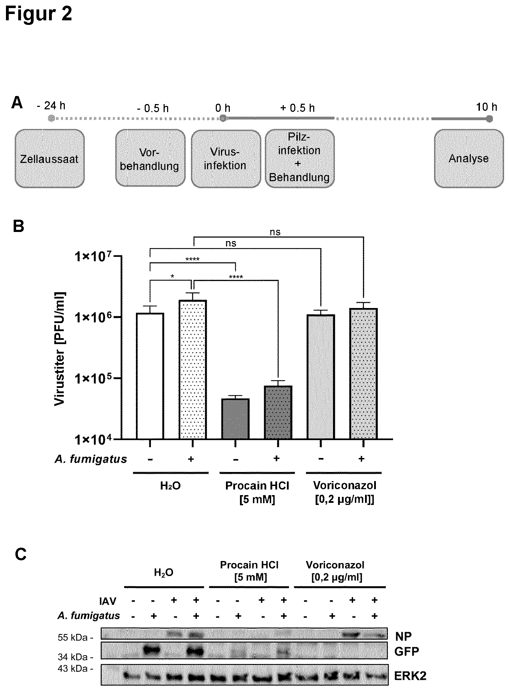

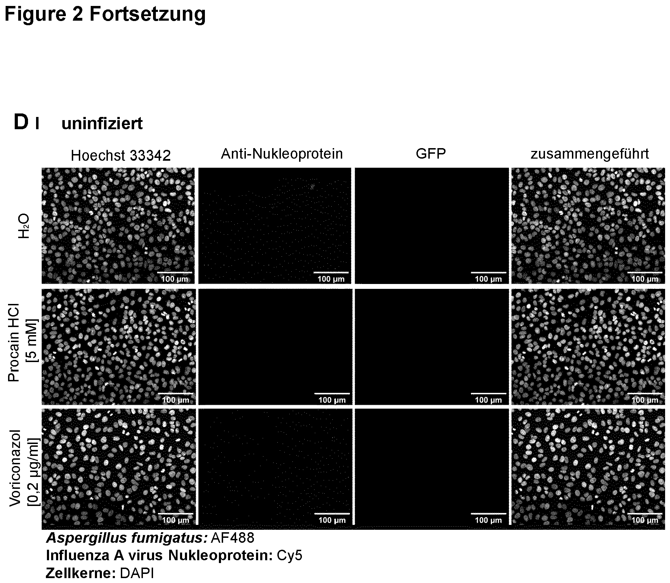

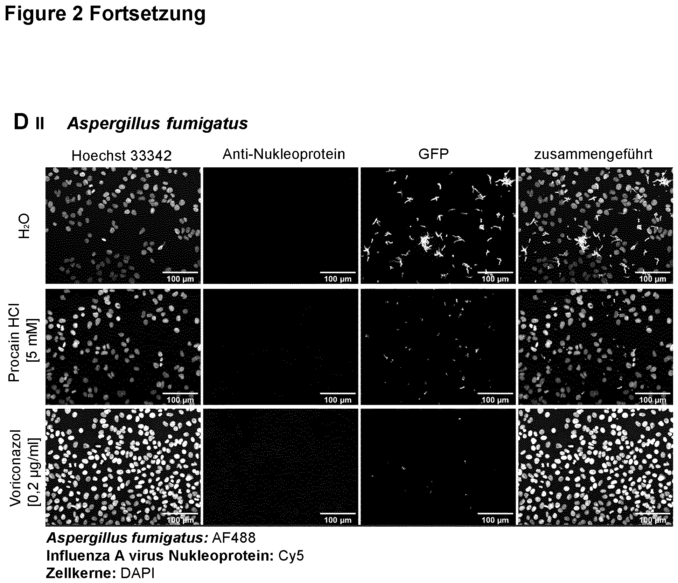

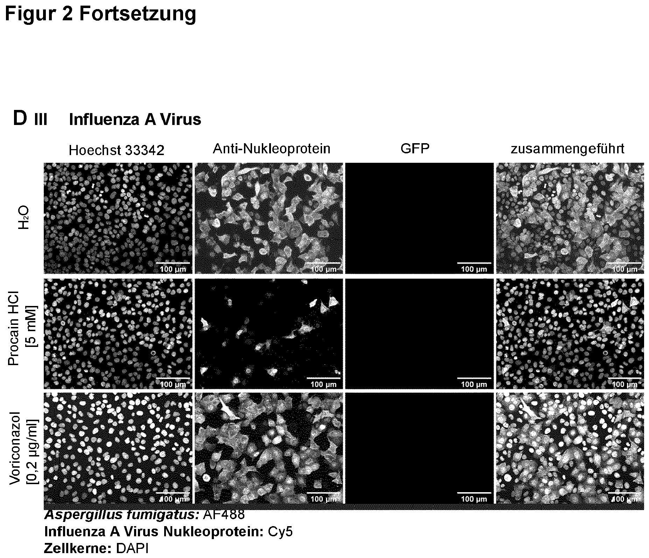

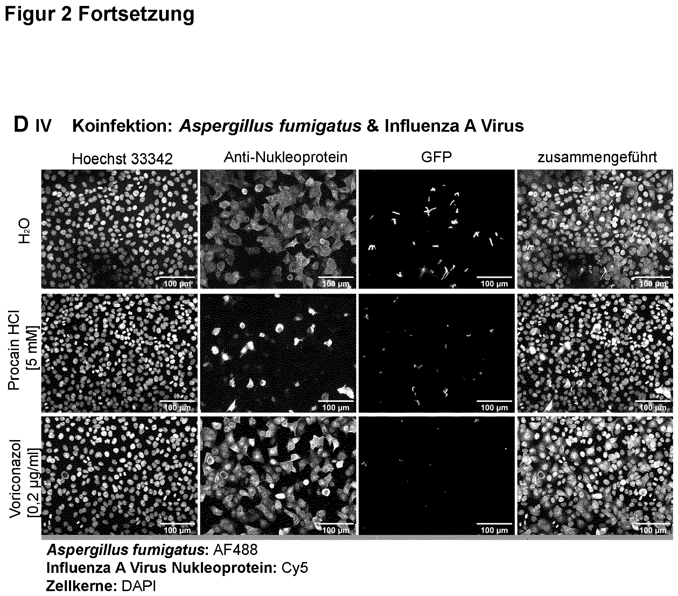

**Figur 2****:** Die Erregerbelastung von Influenzaviren und *Aspergillus fumigatus* ist in *in vitro-Assays* in Gegenwart von procainhaltigen Wirkstoffen (z.B. Procain HCl) reduziert.
   (A) Ablauf des Behandlungsverfahrens: Die humanen Lungenepithelzellen (A549) [0,5*10⁵ Zellen/Well] wurden in DMEM (10 % FCS) auf Deckgläschen in 24 Well-Platten ausgesät und für 23,5 h bei 37 °C, 5 % CO₂ inkubiert. Die Zellen wurden 30 min vor der Infektion mit den angegebenen Konzentrationen von Procain HCl oder Voriconazol in DMEM/BA (0,2 % BSA, 1 mM MgCl₂ und 0,9 mM CaCl₂) vorbehandelt und anschließend 30 min mit dem Influenzavirus A/Puerto Rico/8/34 [0,5 Multiplizität der Infektion (MOI)] in PBS/BA (0,2 % BSA, 1 mM MgCl₂ und 0,9 mM CaCl₂) infiziert. Danach wurde das Inokulum entfernt, Zellen mit PBS gewaschen und Konidien von *Aspergillus fumigatus* [10 MOI] in Gegenwart der angegebenen Substanzen in DMEM/BA/Trypsin (0,2 % BSA, 1 mM MgCl₂ und 0,9 mM CaCl₂, 167 ng ml⁻¹ Trypsin-TPCK) zugegeben. Anschließend wurden die Zellen für 10 h bei 37 °C, 5 % CO₂ inkubiert. (B) Überstände infizierter Zellen wurden gesammelt und virale Titer (*plaque-forming units* (PFU)/ml) mittels Standard-Plaque-Titration untersucht. (C) Die Expression von viralem Nukleoprotein, GFP des *Aspergillus fumigatus* Stammes und dem zellulären Protein ERK2 (*extracellular signal-regulated kinase 2)* wurden in Western Blot-Analysen visualisiert. (D _{I-IV}) Die Zellen wurden fixiert (3,7 % Formaldehyd in PBS, 30 min, Raumtemperatur) und für Immunfluoreszenzuntersuchungen vorbereitet. Das Influenzavirus-Nukleoprotein wurde durch einen Anti-Influenza-Virus-Nukleoprotein-Antikörper (BioRad) und AlexaFluor647 (Invitrogen) visualisiert. Die Zellkerne wurden mit Bisbenzimid-H-33342-Trihydrochlorid (Hoechst-33342; Merck), verdünnt 1:1000 in PBS (3 % normales Ziegenserum), angefärbt. Das Vorhandensein von *Aspergillus fumigatus* konnte durch die interne GFP-Expression (GFP) sichtbar gemacht werden. Die Proben wurden mit Fluoreszenz-Färbelösung (Agilent, Santa Clara, CA, USA) auf Objektträgern aufgebracht. Die Bildaufnahme und -bearbeitung erfolgte mit einem Axio Observer.Z1 Mikroskop (Zeiss, Jena, Deutschland) und der Software Fiji V 1.52b (ImageJ). Dargestellt sind die Aufnahmen der einzelnen Kanäle sowie deren Überlagerung (zusammengeführt). Die Daten stellen den Mittelwert + SD von vier unabhängigen Experimenten dar, einschließlich zweier technischer Proben (B) bzw. eine exemplarische Darstellung aus vier Experimenten (C, D _{I-IV}). Die statistische Signifikanz wurde mit der GraphPad Prism Software Version 9.2 unter Verwendung des Mehrfachvergleichstests von One-Way ANOVA mit einer Tukey -Analyse berechnet. P-Werte sind durch Sternchen *p < 0,05 gekennzeichnet.

### DETAILLIERTE BESCHREIBUNG DER ERFINDUNG

Die Erfindung ist auf ein Amin (AM) gemäß der allgemeinen Formel (I) gerichtet. 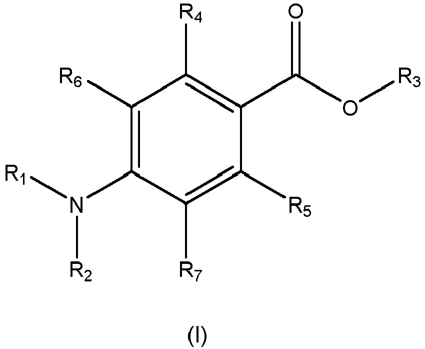 wobei in Formel (I)
R₁ ist H, (C₁₋₁₀)Alkyl, (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₂ ist H, (C₁₋₁₀ Alkyl), (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₃ ist -(CH₂)ₙNR₈R₉;
n ist 1 bis 5, vorzugsweise 1 bis 3, mehr bevorzugt 1 bis 2,
R₈ ist (C₁₋₁₀)Alkyl, vorzugsweise (C₁₋₂)Alkyl,
R₉ ist (C₁₋₁₀)Alkyl, vorzugsweise (C₁₋₂)Alkyl;
R₄ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀)Alkyl, vorzugsweise H, Halogen, (C₁₋₁₀)Alkyl oder -O(C₁₋₁₀) Alkyl, mehr bevorzugt H oder Halogen;
R₅ ist H, Halogen, (C₁₋₁₀)Alkyl, (C₂₋₁₀)Alkenyl, (C₅-C₁₄)Aryl, (C₅-C₁₀)Heteroaryl oder -O-(C₁₋₁₀) Alkyl, vorzugsweise H, Halogen, (C₁₋₁₀)Alkyl oder -O(C₁₋₁₀)Alkyl, mehr bevorzugt H oder Halogen;
R₆ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder -O(C₁₋₁₀)Alkyl, mehr bevorzugt H oder -O(C₁₋₁₀)Alkyl;
R₇ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder -O(C₁₋₁₀)Alkyl, mehr bevorzugt H oder -O-(C₁₋₁₀)Alkyl;
wobei das Amin gemäß Formel (I) optional auch in Form eines Salzes eingesetzt werden kann,
zur Verwendung in der Behandlung von Infektionen mit *Aspergillus fumigatus* und von Superinfektionen mit Influenzaviren und *Aspergillus spp.,* vorzugsweise von Superinfektionen mit Influenzaviren und *Aspergillus spp..*

Vorzugsweise sind die Influenzaviren ausgewählt aus der Gruppe bestehend aus Influenza-A-Viren, Influenza-B-Viren, und Influenza-C-Viren, vorzugsweise Influenza-A-Viren.

*Aspergillus spp.* ist vorzugsweise *Aspergillus fumigatus.*

Unter der Behandlung von Infektionen mit *Aspergillus fumigatus* wird im Rahmen der Erfindung vorzugsweise eine Behandlung von Aspergillose, die durch eine Infektion mit *Aspergillus fumigatus* verursacht wird verstanden.

Unter einer Superinfektion wird im Rahmen dieser Erfindung eine Infektion eines Subjekts (z.B. eines Patienten) zunächst mit einem Erreger verstanden, wobei zeitlich versetzt eine Infektion mit mindestens einem zweiten Erreger erfolgt, bevor die Infektion mit dem ersten Erreger überwunden ist, so dass im Ergebnis zumindest temporär eine simultane Infektion mit dem ersten als auch mit dem zweiten Erreger vorliegt. So kann in diesem Sinne zunächst eine Infektion mit Influenzaviren, vorzugsweise Influenza-A-Viren erfolgen, gefolgt von einer zusätzlichen Infektion mit *Aspergillus spp., vorzugsweise Aspergillus fumigatus* oder zunächst mit *Aspergillus spp.* gefolgt von einer zusätzlichen Infektion mit Influenzaviren, vorzugsweise Influenza-A-Viren, vorzugsweise zunächst eine Infektion mit Influenzaviren, vorzugsweise Influenza-A-Viren erfolgen, gefolgt von einer zusätzlichen Infektion mit *Aspergillus spp..* Vorzugsweise wird im Rahmen der Superinfektion bei der Infektion mit *Aspergillus spp.,* vorzugsweise *Aspergillus fumugatus* eine Aspergillose im infizierten Subjekt ausgelöst. Vorzugsweise wird im Rahmen der Superinfektion bei der Infektion mit Influenzaviren eine Influenza im infizierten Subjekt ausgelöst.

Die Verwendung in der Therapie viraler Erkrankungen, insbesondere von Influenza umfasst die Ausnutzung einer antiviralen Wirkung. Typischerweise hemmen antivirale Wirkstoffe den Entwicklungs- und Vermehrungszyklus, statt die Viren direkt zu attackieren.

Ohne sich auf eine bestimmte theoretische Erklärung festzulegen, wird derzeit davon ausgegangen, dass die beobachtete antivirale Wirkung der erfindungsgemäßen Verbindungen vermutlich vorzugsweise indirekt durch Wechselwirkung mit Stoffwechselwegen des infizierten Organismus vermittelt wird, welche für die Vermehrung des Virus erforderlich sind.

Die Verwendung in der Therapie von Pilzinfektionen umfasst die Ausnutzung einer antimykotischen Wirkung. Typischerweise wirken antimykotische Substanzen zerstörend auf die Zellwand der Pilze.

Ohne sich auf eine bestimmte theoretische Erklärung festzulegen, wird derzeit davon ausgegangen, dass die beobachtete antimykotische Wirkung der erfindungsgemäßen Verbindungen vermutlich vorzugsweise indirekt durch Wechselwirkung mit Stoffwechselwegen des Pilzes vermittelt wird, welche für die Vitalität verantwortlich sind.

In einer Ausführungsform in Formel (I) R₁, R₂, R₃, R₄, R₅, R₆ ist H;
R₃ ist -(CH₂)ₙNR₈R₉;
n ist 1 bis 5, vorzugsweise 1 bis 3, mehr bevorzugt 1 bis 2,
R₈ ist (C₁₋₁₀)Alkyl, vorzugsweise (C₁₋₂)Alkyl,
R₉ ist (C₁₋₁₀)Alkyl, vorzugsweise (C₁₋₂)Alkyl;

In der vorliegenden Erfindung bedeuten Definitionen wie (C₁₋₁₀) Alkyl, wie beispielsweise für den Rest R₁ von Formel (I) definiert, dass dieser Substituent (Rest) ein gesättigter Alkylrest mit einer Kohlenstoffanzahl von 1 bis 10 ist. Der Alkylrest kann sowohl linear als auch verzweigt sowie gegebenenfalls zyklisch sein. Alkylreste, die sowohl eine zyklische als auch eine lineare Komponente aufweisen, fallen ebenfalls unter diese Definition. Gleiches gilt für andere Alkylreste, wie beispielsweise ein C₁₋₂ Alkylrest. Gegebenenfalls können Alkylreste auch mit funktionellen Gruppen wie Amino, Hydroxy, Halogen, Aryl oder Heteroaryl einfach oder mehrfach substituiert sein. Soweit nicht anders angegeben, weisen Alkylreste vorzugsweise keine funktionellen Gruppen als Substituenten auf. Beispiele für Alkylreste sind Methyl, Ethyl, n-Propyl, n-Butyl, n-Pentyl, n-Hexyl, n-Heptyl, n-Octyl, n-Nonyl, iso-Propyl (auch 2-Propyl oder 1-Methylethyl genannt), iso-Butyl, tert-Butyl, sec-Pentyl, neo-Pentyl, 1,2-Dimethylpropyl, iso-Amyl, iso-Hexyl, iso-Heptyl.

In der vorliegenden Erfindung bedeuten Definitionen wie C₂₋₁₀ Alkenyl, wie beispielsweise für den Rest R₁ von Formel (I) definiert, dass dieser Substituent (Rest) ein Alkenylrest mit einer Kohlenstoffanzahl von 2 bis 10 ist, der mindestens eine ungesättigte Kohlenstoff-Kohlenstoffbindung aufweist. Der Alkenylrest kann sowohl linear als auch verzweigt sowie gegebenenfalls zyklisch sein. Alkenylreste, die sowohl eine zyklische als auch eine lineare Komponente aufweisen, fallen ebenfalls unter diese Definition. Gleiches gilt für andere Alkenylreste, wie beispielsweise ein C₂₋₄ Alkenylrest. Gegebenenfalls können Alkenylreste auch mit funktionellen Gruppen wie Amino, Hydroxy, Halogen, Aryl-oder Heteroaryl einfach oder mehrfach substituiert sein. Vorzugsweise weisen Alkenylreste keine weiteren funktionellen Gruppen als Substituenten auf. Beispiele für Alkenylreste sind Vinyl, 1-Propenyl, 2-Propenyl, 1-Butenyl, 2-Butenyl, 3-Butenyl, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, 1-Heptenyl, 3-Heptenyl, 4-Heptenyl, 1-Octenyl, 3-Octenyl, 5-Octenyl, 1-Nonenyl, 2,-Nonenyl.

In der vorliegenden Erfindung bedeutet die Definition Aryl, dass es sich um einen aromatischen oder heteroaromatischen Rest handelt. Ein aromatischer Rest ist ein aromatischer cyclischer Kohlenwasserstoff, der aus einem Ring oder einem Ringsystem aus mehreren kondensierten Ringen bestehen kann. Der aromatische Rest kann beispielsweise monocyclisch, bicyclisch oder tricyclisch sein. Vorzugsweise bildet der monocyclische aromatische Rest einen 5- oder 6-gliedrigen Ring. Vorzugsweise bildet der bicyclische aromatische Ring einen 9- oder 10-gliedrigen Ring. Vorzugsweise bildet der tricyclische aromatische Ring einen 13- oder 14-gliedrigen Ring. Eine Defintion wie (C₅-C₁₄)Aryl bedeutet, dass die Arylgruppe 5 bis 14 Kohlenstoffatome umfasst. Die Arylgruppe enthält vorzugsweise 3 bis 14, mehr bevorzugt 4 bis 6 Kohlenstoffatome. Gegebenenfalls können Arylreste auch mit funktionellen Gruppen wie Alkyl, Alkenyl, Amino, Cyano, -CF₃, Hydroxy, Halogen, Aryl oder Heteroaryl einfach oder mehrfach substituiert sein, vorzugsweise weisen die Arylreste keine weiteren Substituenten auf. Beispiele für aromatische Reste sind Phenyl, und Naphtyl.

In der vorliegenden Erfindung bedeutet die Definition Heteroaryl, dass es sich um einen heteroaromatischen Rest handelt. Heteroaromatischer Ring bedeutet, dass in einem aromatischen Rest, wie vorstehend definiert, dessen Ringsystem durch Kohlenstoffatome gebildet wird, ein oder mehrere dieser Kohlenstoffatome durch Heteroatome wie O, N oder S ersetzt werden. Eine Defintion wie (C₅-C₁₀)Heteroaryl basiert auf der entsprechenden Defintion für die Arylgruppe, und bedeutet dass die Heteroarylgruppe 5 bis 10 Atome im Ring aufweist. Allerdings sind wie oben definiert, ein oder mehrere Kohlenstoffatome durch Heteroatome ersetzt. Das heißt, dass die (C₅-C₁₀)Heteroarylgruppe, 5 bis 10 Atome im Ring aufweist, von denen aber nicht alle Kohlenstoffatome sind. Daher wäre beispielsweise Furanyl eine C₅-Heteroarylgruppe. Gegebenenfalls können Heteroarylreste auch mit funktionellen Gruppen, wie Alkyl, Alkenyl, Amino, Cyano, -CF₃, Hydroxy, Halogen, Aryl oder Heteroaryl einfach oder mehrfach substituiert sein, vorzugsweise weisen die Heteroarylreste keine weiteren Substituenten auf. Beispiele für heteroaromatische Reste, die in der vorliegenden Erfindung unter die Definition Aryl fallen, sind Furanyl, Thienyl, Oxazolyl, Pyrazolyl, Pyridyl und Indolyl.

In der vorliegenden Erfindung bedeutet die Definition Halogen, wie beispielsweise vorstehend für den Rest R₄ für Formel (I) definiert, dass es sich um einen Chlor-, Brom-, Iod- oder Fluorsubstituenten handelt. Vorzugsweise handelt es sich um einen Chlor- oder Fluorsubstituenten.

Vorzugsweise ist das Amin (AM) gemäß der allgemeinen Formel (I) ausgewählt aus der Gruppe bestehend aus 4-Aminobenzoesäure-2-(*N*,*N*-diethylamino-)ethylester (Procain), 4-Aminobenzoesäureethylester (Benzocain), 2-(Diethylamino)ethyl-4-amino-2-chlorbenzoat (Chlorprocain), 4-Amino-3-butoxybenzoesäure-2-diethylaminoethylester (Oxybuprocain), (2-(Dimethylamino)ethyl)-4-(butylamino)benzoat (Tetracain), mehr bevorzugt 4-Aminobenzoesäure-2-(*N*,*N*-diethylamino-)ethylester (Procain).

Die Amine (AM) gemäß der allgemeinen Formel (I) und die oben konkret bezeichneten Amine (AM) sind als Lokalanästhetika in medizinischer Verwendung und entsprechend kommerziell erhältlich.

Weiterhin bezieht sich die Erfindung auf ein Kohlensäure-Addukt (KA) umfassend mindestens ein Strukturelement gemäß der allgemeinen Formel
(II), (III) und/oder (IV) 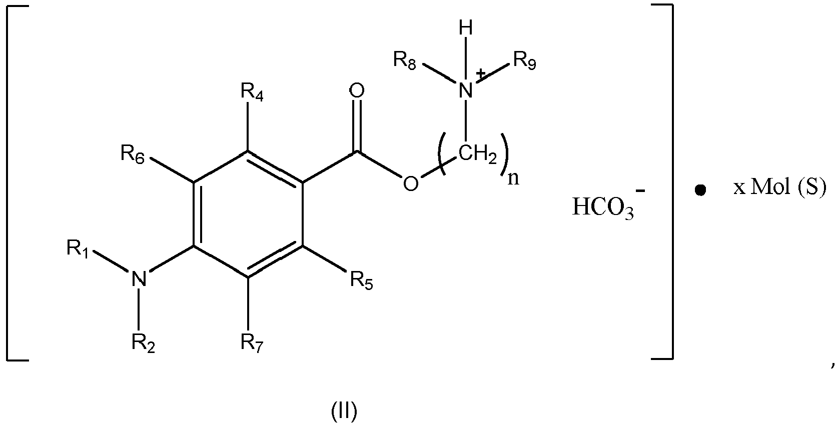 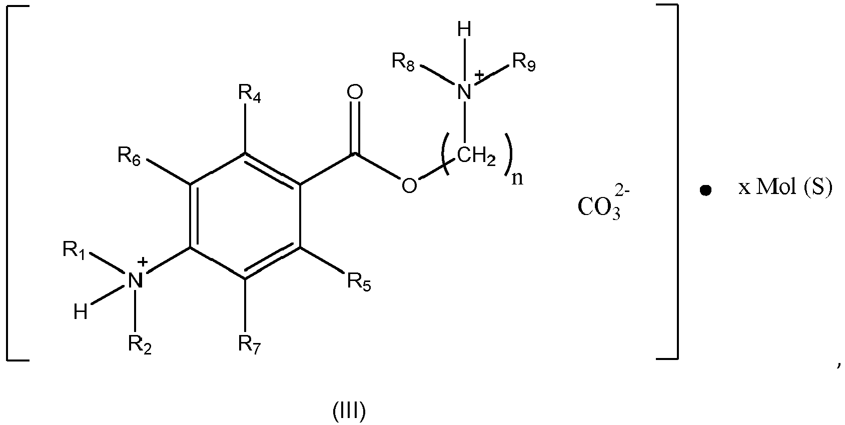 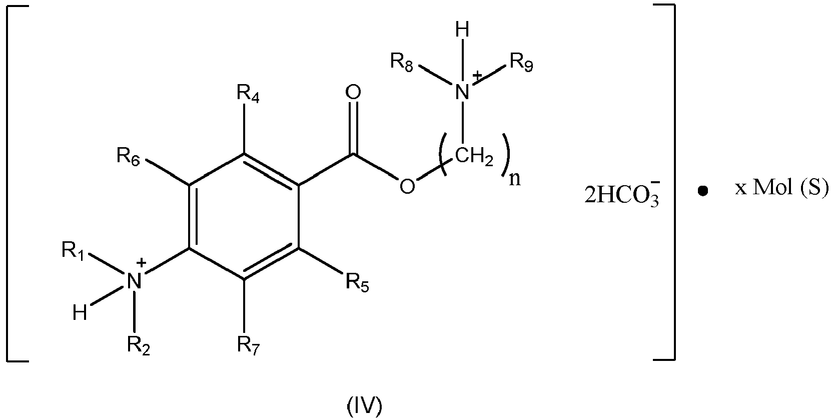 wobei in Formel (II), (III), und (IV)
R₁ ist H, (C₁₋₁₀)Alkyl, (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₂ ist H, (C₁₋₁₀ Alkyl), (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₃ ist -(CH₂)ₙNR₈R₉;
n ist 1 bis 5, vorzugsweise 1 bis 3, mehr bevorzugt 1 bis 2,
R₈ ist (C₁₋₁₀)Alkyl, vorzugsweise (C₁₋₂)Alkyl,
R₉ ist (C₁₋₁₀)Alkyl, vorzugsweise (C₁₋₂)Alkyl;
R₄ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀)Alkyl, vorzugsweise H, Halogen, (C₁₋₁₀)Alkyl oder -O(C₁₋₁₀) Alkyl, mehr bevorzugt H oder Halogen;
R₅ ist H, Halogen, (C₁₋₁₀)Alkyl, (C₂₋₁₀)Alkenyl, (C₅-C₁₄)Aryl, (C₅-C₁₀)Heteroaryl oder -O-(C₁₋₁₀) Alkyl, vorzugsweise H, Halogen, (C₁₋₁₀)Alkyl oder -O(C₁₋₁₀)Alkyl, mehr bevorzugt H oder Halogen;
R₆ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder -O(C₁₋₁₀)Alkyl, mehr bevorzugt H oder -O(C₁₋₁₀)Alkyl;
R₇ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder -O(C₁₋₁₀)Alkyl, mehr bevorzugt H oder -O(C₁₋₁₀)Alkyl;
x is 0,5 bis 30;
(S) ist ein Salz;
zur Verwendung in der Behandlung von Infektionen mit *Aspergillus fumigatus* und von Superinfektionen mit Influenzaviren und *Aspergillus spp.,* vorzugsweise von Superinfektionen mit Influenzaviren und *Aspergillus spp..*

In einer Ausführungsform in Formel (I) R₁, R₂, R₃, R₄, R₅, R₆ is H;
R₃ ist -(CH₂)ₙNR₈R₉;
n ist 1 bis 5, vorzugsweise 1 bis 3, mehr bevorzugt 1 bis 2,
R₈ ist (C₁₋₁₀)Alkyl, vorzugsweise (C₁₋₂)Alkyl,
R₉ ist (C₁₋₁₀)Alkyl, vorzugsweise (C₁₋₂)Alkyl;
Das Salz (S) umfasst mindestens ein Kation ausgewählt aus Na⁺, K⁺, Li⁺, Mg²⁺, Zn²⁺, Fe²⁺, Fe³⁺ und Mn²⁺, vorzugsweise Na⁺. Weiterhin umfasst das Salz (S) mindestens ein Anion ausgewählt aus Cl⁻, Br⁻, J⁻, F⁻, SO₄²⁻, SO₃²⁻, HSO₄⁻ HSO₃⁻, ⁻HCO₃⁻, CO₃²⁻, PO₄³⁻, HPO₄²⁻, H₂PO₄⁻, SiO₄⁴⁻, AlO₂⁻, SiO₃⁻ und /oder [AlO₂)₁₂SiO₂)₂]²⁻, vorzugsweise Cl⁻ und Br⁻, besonders bevorzugt Cl⁻.

Die Herstellung des Kohlensäure-Addukts (KA) ist auch offenbart in WO2006/007835, DE 10 2013 015 035 A1 und WO2019/048590 auf die hier Bezug genommen werden soll.

Weiterhin betrifft die Erfindung ein Kohlensäure-Addukt (KA) umfassend Kohlensäure, mindestens ein Amin (AM) gemäß der allgemeinen Formel (I) und mindestens ein Salz (S), 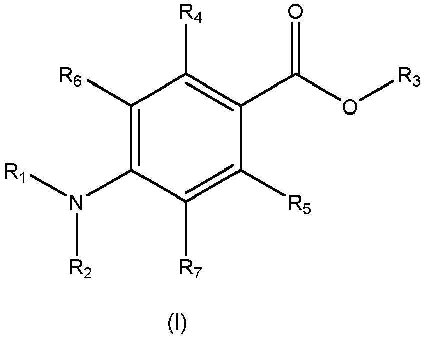 wobei in Formel (I)
R₁ ist H, (C₁₋₁₀)Alkyl, (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₂ ist H, (C₁₋₁₀ Alkyl), (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₃ ist -(CH₂)ₙNR₈R₉;
n ist 1 bis 5, vorzugsweise 1 bis 3, mehr bevorzugt 1 bis 2,
R₈ ist (C₁₋₁₀)Alkyl, vorzugsweise (C₁₋₂)Alkyl,
R₉ ist (C₁₋₁₀)Alkyl, vorzugsweise (C₁₋₂)Alkyl;
R₄ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀)Alkyl, vorzugsweise H, Halogen, (C₁₋₁₀)Alkyl oder -O(C₁₋₁₀) Alkyl, mehr bevorzugt H oder Halogen;
R₅ ist H, Halogen, (C₁₋₁₀)Alkyl, (C₂₋₁₀)Alkenyl, (C₅-C₁₄)Aryl, (C₅-C₁₀)Heteroaryl oder -O-(C₁₋₁₀) Alkyl, vorzugsweise H, Halogen, (C₁₋₁₀)Alkyl oder -O(C₁₋₁₀)Alkyl, mehr bevorzugt H oder Halogen;
R₆ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder -O(C₁₋₁₀)Alkyl, mehr bevorzugt H oder -O(C₁₋₁₀)Alkyl;
R₇ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder -O(C₁₋₁₀)Alkyl, mehr bevorzugt H oder -O(C₁₋₁₀)Alkyl;
wobei das mindestens eine Amin gemäß Formel (I) optional auch in Form eines Salzes eingesetzt werden kann;
zur Verwendung in der Behandlung von Infektionen mit *Aspergillus fumigatus* und von Superinfektionen mit Influenzaviren und *Aspergillus spp..*

In einer Ausführungsform in Formel (I), R₁, R₂, R₄, R₅, R₆ ist H;
R₃ ist -(CH₂)ₙNR₈R₉;
n ist 1 bis 5, vorzugsweise 1 bis 3, mehr bevorzugt 1 bis 2,
R₈ ist (C₁₋₁₀)Alkyl, vorzugsweise (C₁₋₂)Alkyl,
R₉ ist (C₁₋₁₀)Alkyl, vorzugsweise (C₁₋₂)Alkyl;

Das Salz (S) kann beispielsweise, durch eine Säure-Base-Reaktion der Base (BA), bei Durchführung von Schritt b) mit der an das Amin (AM) addierten Säure gebildet werden, falls ein Säureadditionssalz des Amins (AM) eingesetzt wird. Das Salz (S) kann auch direkt in einem der Schritte a), b) und/oder c) zugesetzt werden. Der direkte Zusatz des Salzes (S) ist bevorzugt, falls das Amin (AM) nicht in der Salzform eingesetzt und/oder der Schritt b) nicht durchgeführt wird.

Vorzugsweise bleibt das Kohlensäure-Addukt (KA) bei Lagerung bei einer Temperatur von 2 bis 10 °C für mindestens 12 Monate, mehr bevorzugt für mindestens 13 Monate, noch mehr bevorzugt für mindestens 20 Monate, besonders bevorzugt für mindestens 23 Monate, und ganz besonders bevorzugt für mindestens 27 Monate stabil.

Das Kohlensäure-Addukt (KA) gilt nicht mehr als stabil, wenn mit IR-Spektroskopie, insbesondere im Feststoff des Kohlensäure-Adduktes (KA), die spezifischen Banden des mindestens einen Amins (AM) detektiert werden können. Die spezifischen IR-Banden des Amins sind diejenigen Banden, die auch bei der IR-spektroskopischen Untersuchung des reinen Amins (AM) detektiert werden. Solange das Amin (AM) gebunden im stabilen Kohlensäure-Addukt (KA) vorliegt, werden die spezifischen IR-Banden des Amins (AM) nicht detektiert.

Der Verlust der Stabilität kann in einzelnen Ausführungsformen auch mit einer Erhöhung des pH-Wertes, oder mit der Messung von zwei Schmelz-/Zersetzungsbereichen, d.h. einem Bereich der dem Amin (KA) entspricht und einem Bereich der dem Kohlensäure-Addukt (KA) entspricht einhergehen. Bei dem Kohlensäure-Addukt, das durch zumindest teilweise Zersetzung in das Amin (AM) und CO₂ und/oder Wasser, instabil geworden ist, kann es auch zu einer Änderung des Lösungsverhaltens kommen. Das instabil gewordene Kohlensäure-Addukt (KA) kann sich als schwerlöslicher erweisen oder zumindest teilweise unvollständig gelöst werden.

Das Kohlensäure-Addukt (KA) ist herstellbar nach einem Verfahren umfassend die Schritte a), optional b), c), d) und e).

In Schritt a) wird eine Lösung (A) bereitgestellt, die mindestens ein Lösungsmittel und indem mindestens einen Lösungsmittel gelöstes CO₂ umfasst.

Die Lösung (A) umfasst mindestens ein Lösungsmittel und CO₂ in gelöster Form. Unter CO₂ wird im Rahmen dieser Erfindung Kohlendioxid verstanden. Unter CO₂ in gelöster Form werden im Rahmen dieser Erfindung alle Formen des CO₂ verstanden, die dieses beim Lösen eingeht. So ist beispielweise für wässrige Lösungen bekannt, dass das gelöste CO₂ in der Lösung unter anderem im Gleichgewicht als CO₂, als Kohlensäure, als einfach oder zweifach deprotonierte Kohlensäure, das heißt als Hydrogencarbonat oder Carbonat vorliegen kann.

Die Lösung (A) wird erhalten, in dem CO₂ in das mindestens eine Lösungsmittel eingebracht wird. Das CO₂ kann in allen dem Fachmann bekannten und geeigneten Formen und in das Lösungsmittel eingebracht werden. Vorzugsweise wird gasförmiges oder gefrorenes CO₂ in Form von Trockeneis, mehr bevorzugt gasförmiges CO₂ in das Lösungsmittel eingebracht. Vorzugsweise hat das gasförmige CO₂ oder gefrorene CO₂ mindestens Lebensmittelqualität, mehr bevorzugt eine Qualität, die für pharmazeutische Zwecke geeignet ist. Das verwendete gasförmige und/oder gefrorene CO₂ hat vorzugsweise einen Reinheitsgrad von mindestens 99,5%, noch mehr bevorzugt 99,9%.

Vorzugsweise bedeutet Lebensmittelqualität im Rahmen dieser Erfindung, dass die einschlägigen Vorschriften des deutschen und europäischen Lebensmittelrechts erfüllt werden. Diese umfassen vorzugsweise die VO (EG) Nr. 852/2004 und VO (EG) Nr. 178/2002 sowie die VO (EG) Nr. 1333/2008 und die VO (EU) 231/2012.

Vorzugsweise bedeutet pharmazeutische Qualität, dass die einschlägigen Vorschriften des Europäischen Arzneimittelbuchs (Ph. Eur.) erfüllt werden.

Das CO₂ kann auch unter Druck eingebracht werden, insbesondere wenn gasförmiges CO₂ in die Lösung eingebracht wird. Einbringen unter Druck bedeutet in diesem Zusammenhang, dass ein Druck größer dem Atmosphärendruck, vorzugsweise größer 1,01325 bar, verwendet wird. Zu diesem Zweck kann das Einbringen des CO₂ in das Lösungsmittel in einem Behälter erfolgen, der das Lösungsmittel derartig von der Umgebung isoliert, dass in dem Behälter ein Druck erzeugt werden kann, insbesondere über das Zuleiten des CO₂, der über dem atmosphärischen Druck, vorzugsweise über 1,01325 bar liegt. Das Einbringen des CO₂ in die Lösung, insbesondere in gasförmiger Form, kann in einem Schritt oder in Intervallen erfolgen.

Der Fachmann kann in Schritt a) jedes geeignete Lösungsmittel einsetzen. Vorzugsweise wird als Lösungsmittel ein polar-protisches Lösungsmittel eingesetzt, mehr bevorzugt ist das Lösungsmittel Wasser. Das Lösungsmittel kann je nach beabsichtigtem Verwendungszweck des Kohlensäure-Addukts (KA) in verschiedenen Reinheitsgraden eingesetzt werden. Beispielsweise kann Wasser mit dem Reinheitsgrad *"Aqua ad iniectabilia"* verwendet werden, falls das Kohlensäure-Addukt (KA) für pharmazeutisch-medizinische Zwecke eingesetzt werden soll.

Schritt a) kann den Teilschritt a1) umfassen, wobei das Lösungsmittel, vorzugsweise vor dem Einbringen des CO₂, auf 3 bis 8 °C, vorzugsweise auf 5 °C gekühlt wird. Das Kühlen kann mittels allen dem Fachmann bekannten und als geeignet identifizierten Methoden erfolgen. Beispielsweise kann das Kühlen durch Verwahren des Lösungsmittels für eine ausreichend lange Zeit in einem Kühlschrank erfolgen, bis das Lösungsmittel die Zieltemperatur aufweist. Ebenso kann beispielsweise eine externe Kühlung verwendet werden.

Schritt a) kann den Teilschritt a2) umfassen, wobei das CO₂ in das Lösungsmittel eingebracht wird, vorzugsweise bis zum Erreichen einer Sättigungskonzentration von 3 bis 10 g/l, mehr bevorzugt bis zu einer Sättigungskonzentration von 4,5 bis 7,5 g/l bezogen auf das Gesamtvolumen der Lösung. Vorzugsweise beträgt der pH-Wert der Lösung nach der Sättigung mit CO₂ ≤ 3,0 bis ≤ 6,0, noch mehr bevorzugt ≤ 4,3 bis ≤ 4,8. Vorzugsweise wird das CO₂ in Teilschritt a2) unter Druck gelöst, wobei der Druck 1,5 bis 10 bar, mehr bevorzugt 1,9 bis 7 bar, noch mehr bevorzugt 2 bis 5 bar beträgt.

Schritt a) kann den Teilschritt a3) umfassen, wobei die vorzugsweise in Teilschritt a2) erhaltene Lösung (A) bei 1 bis 10 °C vorzugsweise für mindestens 30 min, mehr bevorzugt für mindestens 50 min, noch mehr bevorzugt für mindestens 60 min; bis höchstens 5 Tage (120 h) gelagert wird. Vorzugsweise wird die vorzugsweise in Teilschritt a2) erhaltene Lösung (A) bei 3 bis 8 °C für mindestens 30 min, mehr bevorzugt für mindestens 50 min, noch mehr bevorzugt für mindestens 60 min; bis höchstens 5 Tage (120 h) gelagert.

Vorzugsweise umfasst Schritt a) alle Teilschritte a1), a2) und a3).

Vorzugsweise werden die Teilschritte a1), a2) und a3) in der Reihenfolge a2) folgt auf a1) und a3) folgt auf a2) durchgeführt.

Optional kann der Schritt b) durchgeführt werden, wobei die Base (BA), die nicht dem Amin (AM) entspricht, in der Lösung (A), unter Erhalt der Lösung (A1) aufgelöst wird. Vorzugsweise ist die Base (BA) ein Hydrogencarbonat oder ein Carbonat, mehr bevorzugt ein Hydrogencarbonat, noch mehr bevorzugt Natriumhydrogencarbonat.

In Schritt c) bzw. c1) wird das mindestens eine Amin (AM) in der Lösung (A) oder (A1) unter Erhalt der Lösung (B) gelöst.

Das mindestens eine Amin (AM), wie vorstehend definiert, kann in Schritt c) sowohl in neutraler Form als auch in Form eines Salzes eingesetzt werden. Optional kann das mindestens eine Amin (AM) auch als Mischung der neutralen Form des Amins (AM) mit der Salzform des Amins (AM) eingesetzt werden. Daher kann das mindestens eine Amin (AM), das neutrale Amin (AM) und/oder die Salzform des mindestens einen Amins (AM) umfassen. Vorzugsweise handelt es sich bei der Salzform des mindestens einen Amins (AM) um ein Säureadditionssalz, vorzugsweise ist das Säureadditionsalz ein Hydrochlorid, Hydrobromid, Hydroiodid, Hydrogensulfat, Hydrogensulfit, Hydrogenphosphat, Hydromesylat, Hydrotosylat, Hydroacetat, Hydroformiat, Hydropropanoat, Hydromalonat, Hydrosuccinat, Hydrofumarat, Hydroxalat, Hydrotartrat, Hydrocitrat, Hydromaleat, mehr bevorzugt ein Hydrochlorid oder Hydrobromid, noch mehr bevorzugt ein Hydrochlorid des mindestens einen Amins (AM).

Vorzugsweise beträgt die Konzentration des Amins (AM) in Lösung (B) 0,01 bis 0,25 g/ml, vorzugsweise 0,03 bis 0,20 g/ml, mehr bevorzugt 0,08 bis 0,15 g/ml.

Schritt c) kann den Teilschritt c2) umfassen, wobei das mindestens eine Amin (AM) in Lösung (A), oder bei Durchführung von Schritt b), in Lösung (A1) unter Erhalt der Lösung (B) aufgelöst wird.

In einer möglichen Ausführungsform beträgt das Verhältnis des Amins (AM) zur Base (BA), bei Durchführung von Schritt b), in Lösung (B) 2:1 bis 5:1, mehr bevorzugt 3:1 bis 4:1, noch mehr bevorzugt 3,23:1 bis 3,26:1 [g/g]].

In einer weiteren möglichen Ausführungsform beträgt das molare Verhältnis des Amins (AM) zu den Basenäquivalenten der Base (BA), bei Durchführung von Schritt b), in Lösung (B) 0,8:1 bis 1,5:1, vorzugsweise 1,2:1, mehr bevorzugt 1:1. Basenäquivalente bedeutet in diesem Zusammenhang, dass bei Verwendung einer einwertigen Base, wie zum Beispiel NaHCO₃ das molare Verhältnis der Base (BA) zum Amin (AM dem vorstehend angegebenen Verhältnis entspricht. Bei Verwendung einer zweiwertigen Base (BA), wie zum Beispiel Na₂CO₃, ist bezogen auf die Stoffmenge in Mol der Base (BA) relativ zur Verwendung einer einwertigen Base nur die Hälfte der Basenmenge erforderlich um die gleiche Menge an Basenäquivalenten einzubringen. So werden zum Beispiel bei einem Verhältnis von 1:1, bei Verwendung von 10 mmol Amin (AM), 10 mmol NaHCO₃ benötigt, aber nur 5 mmol Na₂CO₃.

In einer weiteren Ausführungsform wird Schritt b) ausgeführt und in Teilschritt c1) das Amin (AM) in Form des Säureadditionssalzes zugesetzt, wobei das Amin (AM) mit der an ihm gebundenen Säure, in einer derartigen Menge zugesetzt wird, dass die an das Amin (AM) gebundene Säure, die Base (BA) soweit zu neutralisieren vermag, dass die Lösung (B) einen pH-Wert von 6 bis 8 annimmt.

Schritt c) kann den Teilschritt c2) umfassen, wobei Lösung (A) zur Lösung (B) unter Erhalt der Lösung (B1) gegeben wird.

Vorzugsweise beträgt die Konzentration des Amins (AM) in Lösung (B1) 0,01 bis 0,25 g/ml, vorzugsweise 0,03 bis 0,20 g/ml, mehr bevorzugt 0,08 bis 0,15 g/ml.

Schritt c) kann den Teilschritt c3) umfassen, wobei die Lösung (B) oder bei Durchführung von Teilschritt c2), die Lösung (B1) mit CO₂ angereichert wird. Vorzugsweise wird die Lösung (B) mit 2,5 g/l bis 9 g/l, mehr bevorzugt mit 5 bis 7,5 g/l CO₂ angereichert.

Schritt c) kann den Teilschritt c4) umfassen, wobei die Lösung (B) oder, bei Durchführung von Teilschritt c2), die Lösung (B1) bei 1 bis 10°C, vorzugsweise 3 bis 8 °C, für mindestens 1 h vorzugsweise 24 h bis 120 h, noch mehr bevorzugt 24 bis 72 h gelagert wird.

Schritt c) kann den Teilschritt c5) umfassen, wobei die Lösung (B) oder bei Durchführung von Teilschritt b2) die Lösung (B1) mit CO₂ vorzugsweise auf eine Gesamtkonzentration von mindestens 6 g/l, mehr bevorzugt mindestens 10 g/l, noch mehr bevorzugt mindestens 12 g/l, ganz besonders bevorzugt mindestens 14 g/l und am meisten bevorzugt mindestens 15 g/l angereichert wird. Vorzugsweise werden in Teilschritt c5) weitere 0,4 bis 4,7 g/l, mehr bevorzugt 1 bis 3,5 g/l CO₂ bis zum Erreichen der benötigten Gesamtkonzentration in die Lösung (B) oder (B1) eingebracht bzw. gelöst.

Der Begriff "Gesamtkonzentration" bezieht sich hier auf die Gesamtkonzentration an gelöstem CO₂ in der Lösung (B) oder (B1), einschließlich dem CO₂, das im Kohlensäure-Addukt (KA) gebunden ist. Die Gesamtkonzentration ergibt sich additiv aus der Gewichtszunahme der Lösung durch das zugeführte CO₂ in allen vorangegangenen Anreicherungsschritten a2) und/oder c3), soweit ausgeführt, und c5), ohne Berücksichtigung von CO₂, das optional in Form von Hydrogencarbonat oder Carbonat als Base (BA) der Lösung zugesetzt wird.

Das Anreichern der Lösung (B) oder der Lösung (B1) in Teilschritt c5) mit CO₂ auf die benötigte Gesamtkonzentration kann bei einem Druck von 2,5 bis 10 bar, vorzugsweise von 4 bis 10 bar, mehr bevorzugt von 5 bis 10 bar, noch mehr bevorzugt bei 6 bis 10 bar, ganz besonders bevorzugt bei 6,5 bis 10 bar durchgeführt werden. Vorzugsweise weist die Lösung (B) oder (B1), beim Anreichern mit CO₂ in Teilschritt c5) eine Temperatur von 3 bis 8 °C, mehr bevorzugt von 5°C auf.

Die Anreichung der Lösung (B) oder (B1) in den Teilschritten c3) und c5) kann auf dieselbe Weise erfolgen, wie für Schritt a) beschrieben.

Vorzugsweise beträgt der pH-Wert der Lösung (B) oder, bei Durchführung des Teilschritts c2), der Lösung (B1) nach der Durchführung von Schritt c5) ≤ 7,0.

Vorzugsweise umfasst Schritt c) alle Teilschritte c1), c2), c3), c4) und c5).

Vorzugsweise werden die Teilschritte c1), c2), c3), c4) und c5) in der Reihenfolge c2) folgt auf c1), c3) folgt auf c2), c4) folgt auf c3), c5) folgt auf c4) durchgeführt.

In Schritt d) wird die nach Abschluss des Schrittes c) erhaltene Lösung eingefroren. Vorzugsweise wird in Schritt d) die Lösung (B) oder nach Durchführung des Teilschrittes c2), die Lösung (B1) eingefroren.

Die Lösung, vorzugsweise Lösung (B) oder (B1), die dem Schritt d) unterzogen wird, weist einen CO₂-Gehalt von mindestens 6 g/l, vorzugsweise mindestens 10 g/l, mehr bevorzugt mindestens 12 g/l, noch mehr bevorzugt mindestens 14 g/l und ganz besonders bevorzugt mindestens 15 g/l auf.

Vorzugsweise wird die nach Abschluss des Schrittes c) erhaltene Lösung, vorzugsweise Lösung (B) oder (B1), bei -100 °C bis -20 °C, mehr bevorzugt bei -90 °C bis -30 °C, noch mehr bevorzugt bei -80 bis -40 °C und ganz besonders bevorzugt bei -70 bis -50 °C eingefroren.

Das Einfrieren der nach Abschluss des Schrittes c) erhaltenen Lösung, vorzugsweise Lösung (B) oder (B1), kann grundsätzlich nach allen dem Fachmann bekannten und als geeignet identifizierten Methoden erfolgen. Beispielsweise kann das Einfrieren durch das Überführen der in Schritt c) erhaltenen Lösung in ein geeignetes Gefäß, erfolgen, dass in ein Kühlmedium eintaucht. Vorzugsweise hat das Gefäß eine Kolbenform. Vorzugsweise taucht das Gefäß, in dem sich die in Schritt c) erhaltene Lösung befindet in einem Winkel von 40 ° in das Kühlmedium ein. Das Kühlmedium kann beispielsweise aus einem Lösungsmittel wie Methanol, Ethanol oder Aceton bestehen, das durch Zugabe von Trockeneis, oder geeignete Kühlapparaturen, wie Kryostaten auf die gewünschte Temperatur gebracht wird.

Vorzugsweise erfolgt das Einfrieren bei Atmosphärendruck, mehr bevorzugt bei 1,01325 bar.

Vorzugsweise wird die nach Abschluss des Schrittes c) erhaltene Lösung, vorzugsweise Lösung (B) oder (B1), innerhalb von 0,3 bis 60 Minuten, mehr bevorzugt innerhalb von 1 bis 30 Minuten, noch mehr bevorzugt innerhalb von 1,1 bis 10 Minuten, besonders bevorzugt innerhalb von 1,5 bis 5 Minuten eingefroren.

Die nach Abschluss des Schrittes c) erhaltene Lösung, vorzugsweise Lösung (B) oder (B1), wird vorzugsweise mit einer Kühlrate von 10 bis 100 K/min, mehr bevorzugt mit 20 bis 80 K/min, noch mehr bevorzugt mit 30 bis 70 K/min und besonders bevorzugt mit 40 bis 60 K/min eingefroren.

Vorzugsweise wird das Gefäß in dem sich die nach Abschluss des Schrittes c) erhaltene Lösung, vorzugsweise Lösung (B) oder (B1), während des Einfriervorgangs befindet, im Kühlmedium, mit 10 bis 1000 rpm, mehr bevorzugt mit 50 bis 600 rpm, noch mehr bevorzugt mit 100 bis 400 rpm und besonders bevorzugt mit 200 bis 300 rpm rotiert.

Das Einfrieren kann nach dem Shell-Freeze-Verfahren erfolgen.

In Schritt e) wird die in Schritt d) eingefrorene Lösung, vorzugsweise Lösung (B) oder (B1), bei -100 bis 0 °C für nicht länger als 4 Tage gelagert.

Vorzugsweise wird die in Schritt d) eingefrorene Lösung in Schritt e), vorzugsweise Lösung (B) oder (B1), für 1,5 bis 4 Tage, mehr bevorzugt für 2,5 bis 4 Tage gelagert.

Vorzugsweise wird die in Schritt d) eingefrorene Lösung in Schritt e), vorzugsweise Lösung (B) oder (B1) bei -50 bis 0 °C, mehr bevorzugt bei -30 bis -5 °C, noch mehr bevorzugt bei -25 bis -10 °C, besonders bevorzugt bei -20 bis -15 °C gelagert.

Die Lagerung kann bei der definierten Temperatur grundsätzlich in jeder dem Fachmann bekannten Kühleinrichtung erfolgen. Beispielsweise kann die Lagerung in einem Tiefkühlschrank oder einem Tiefkühlraum durchgeführt werden.

Das Verfahren nach dem das Kohlensäure-Addukt (KA) herstellbar ist, kann einen weiteren Schritt f) umfassen, der nach Schritt e) durchgeführt wird. Dabei wird in Schritt f) die in Schritt e) gelagerte Lösung, vorzugsweise Lösung (B) oder (B1), getrocknet, unter Erhalt von getrocknetem Kohlensäure-Addukt (KA).

Vorzugsweise wird in Schritt f) das Wasser aus der in Schritt e) gelagerten Lösung, vorzugsweise Lösung (B) oder (B1) bis zu einem Restgehalt von < 0,8 Gew.-%, mehr bevorzugt bis zu einem Restgehalt von < 0,1 Gew.-% bezogen auf das Gesamtgewicht des getrockneten Kohlensäure-Adduktes (KA) entfernt.

Vorzugsweise wird in Schritt f) nicht im Kohlensäure-Addukt (KA) gebundenes CO₂ aus der in Schritt e) gelagerten Lösung, vorzugsweise (B) oder (B1), bis zu einem Restgehalt von < 0,8 Gew.-%, mehr bevorzugt bis zu einem Restgehalt von < 0,1 Gew.-% bezogen auf das Gesamtgewicht des getrockneten Kohlensäure-Adduktes (KA) entfernt.

Die Trocknung kann mit allem dem Fachmann bekannten und als geeignet identifizierten Methoden durchgeführt werden. Vorzugsweise erfolgt die Trocknung mittels Gefriertrocknung, auch Lyophilisation genannt. Der Schritt d) stellt im Falle der Anwendung des Gefriertrocknungsverfahrens, den Einfrierschritt und Schritt e) den Reifungsschritt dar.

Vorzugsweise beträgt der Druck während des Trocknens 0,01 bis 30 mbar, vorzugsweise 0,02 bis 20 mbar, mehr bevorzugt 0,03 bis 10 mbar, noch mehr bevorzugt 0,03 bis 0,5 mbar und ganz besonders bevorzugt 0,05 bis 0,1 mbar. Vorzugsweise wird der Druck während des gesamten Trocknungsvorgangs beibehalten. Vorzugsweise wird der vorstehend definierte Druck während des Trocknens innerhalb von 7 h, mehr bevorzugt innerhalb von 5 h und besonders bevorzugt innerhalb von 4 h ab Evakuierungsbeginn erreicht.

Den Endpunkt der Trocknung kann der Fachmann aus den Temperaturverlaufsaufzeichnungen ermitteln. Vorzugsweise beträgt die Gesamttrocknungszeit in Schritt f) 10 bis 60 h, mehr bevorzugt 30 bis 55 h, besonders bevorzugt 41 bis 52 h. Die Gesamttrocknungszeit ist definiert als die Zeitspanne zwischen dem Abschluss der Lagerung in Schritt e) und der Beendigung der Trocknung in Schritt f).

Vorzugsweise beträgt die Temperatur während der gesamten Trocknung in Schritt f) 0 bis 20 °C, vorzugsweise 4 bis 18 °C, mehr bevorzugt 8 bis 16 °C.

Weiterhin umfasst die Erfindung eine pharmazeutische Zusammensetzung (PZ) umfassend das Amin (AM), oder das Kohlensäure-Addukt (KA) wie oben beschrieben zur Verwendung in der Behandlung von atypischer Pneumonie.

Pharmazeutische Zusammensetzungen, die auch das Kohlensäure-Addukt (KA) enthalten, sind in WO2019/048590 beschrieben.

Unter der pharmazeutischen Zubereitung (PZ) im Rahmen dieser Erfindung wird grundsätzlich eine Zusammensetzung verstanden, die das Amin (AM) oder Kohlensäure-Addukt (KA) umfasst und darüber hinaus, weitere Hilfsstoffe oder Zusätze umfassen kann, die für eine pharmazeutisch-medizinische Verwendung geeignet sind.

Außerdem kann die pharmazeutische Zubereitung (PZ) weitere Basen umfassen, die nicht dem Amin (AM) entsprechen und verschieden von der Base (BA) sein können. Der Fachmann kann die Additive grundsätzlich je nach gewünschtem Verwendungszweck auswählen. Er wird dabei die gewünschte Applikationsform berücksichtigen.

Die pharmazeutische Zubereitung (PZ) kann grundsätzlich in jeder geeigneten Darreichungsform vorliegen. So kann die pharmazeutische Zubereitung (PZ) beispielsweise in Kapselform, als Tablette, als Lösung, als Salbe, Creme, als Gel, als Paste, Umschlagpaste oder wirkstoffhaltiges Pflaster vorliegen.

Die pharmazeutische Zubereitung (PZ) kann grundsätzlich in jeder geeigneten Applikationsform appliziert werden. Der Fachmann wird eine geeignete Darreichungsform entsprechend der beabsichtigten Applikationsform auswählen. Beispielsweise kann die pharmazeutische Zubereitung (PZ) oral, inhalativ, per Injektion, als Pflaster, kutan umfassend mindestens die dermale Anwendung, die Anwendung am Auge, die nasale Anwendung, die rektale Anwendung und die vaginale Anwendung; verabreicht werden.

Bei der Zubereitung der pharmazeutischen Zubereitung (PZ) kann sich der Fachmann grundsätzlich der im Stand der Technik bekannten Methoden bedienen.

Vorzugsweise beträgt die Temperatur der Mischung aus dem Kohlensäure-Addukt (KA) und den verwendeten Hilfsstoffen und gegebenenfalls weiteren Basen während der Zubereitung der pharmazeutischen Zubereitung (PZ) weniger als 60 °C, vorzugsweise weniger als 50 °C, mehr bevorzugt 0 bis 50 °C.

Bei der Zubereitung der pharmazeutischen Zubereitung (PZ), vorzugsweise in Salbenform, kann auch Dispergieren, vorzugsweise mittels eines Salbenbereiters, zum Einsatz kommen. Hierbei wird vorzugsweise eine Drehzahl von < 2000 rpm verwendet.

Das Kohlensäure-Addukt (KA) kann vor der Verarbeitung zu den nachfolgend beschriebenen oralen Darreichungsformen wie Tabletten, Kapseln oder halbfesten Darreichungsformen, alleine oder in der Anwesenheit weiterer Hilfsstoffe oder Basen zu Pulver verrieben werden. Der Fachmann kann sich grundsätzlich der für den jeweiligen Zweck geeigneten und bekannten technischen Mittel bedienen. So können für Zerreibungsschritte beispielsweise Mörser oder ähnlich geeignete Geräte eingesetzt werden. Vorzugsweise wird bei den Verreibungsschritten ein technisches Hilfsmittel eingesetzt, dass die mechanische Belastung des Kohlensäure-Adduktes möglichst gering hält. Vorzugsweise erfolgt das Zerreiben mit einem Mörser.

Das so erhaltene Pulver kann dann zum Beispiel zu Tabletten gepresst oder in handelsübliche Kapseln abgefüllt oder mit geeigneten Hilfsstoffen vermischt und zu halbfesten Darreichungsformen verarbeitet werden.

Eine Ausführungsform der pharmazeutischen Zubereitung (PZ) betrifft eine pharmazeutische Zubereitung (PZ), die das Kohlensäure-Addukt (KA) umfasst und oral appliziert wird. In dieser Ausführungsform wird die pharmazeutische Zubereitung (PZ) vorzugsweise in Kapseln, mehr bevorzugt in Hartgelatine oder Cellulose-Kapseln, besonders bevorzugt in Hartgelatine Kapseln verabreicht. Ebenso kann die pharmazeutische Zubereitung (PZ) in dieser Ausführungsform in Tablettenform appliziert werden.

Vorzugsweise umfasst die pharmazeutische Zubereitung (PZ) in dieser Ausführungsform mindestens ein Hilfsstoff (H), vorzugsweise ausgewählt aus Stärke, insbesondere Maisstärke und/oder Reisstärke, Dextran, Celluloseester und SiO₂.

Zudem kann die pharmazeutische Zubereitung (PZ) in dieser Ausführungsform mindestens eine Base (BA1) umfassen, die nicht dem Amin (AM) entspricht und identisch oder verschieden von der Base (BA) ist. Vorzugsweise ist die Base (BA1) ausgewählt aus NaHCO₃ oder KHCO₃, mehr bevorzugt NaHCO₃.

Vorzugsweise gelten auch für diese Ausführungsform die vorstehend gemachten allgemeinen Angaben zur pharmazeutischen Zubereitung (PZ), insbesondere auch für die Zubereitung der pharmazeutischen Zubereitung (PZ), soweit für diese Ausführungsform technisch anwendbar.

Vorzugsweise umfasst die pharmazeutische Zubereitung (PZ) in dieser Ausführungsform:
a) 1 bis 99 Gew.-%, mehr bevorzugt 15 bis 95 Gew.-% des Kohlensäure-Addukts (KA),
b) 0 bis 60 Gew.-%, mehr bevorzugt 3 bis 50 Gew.-% der Base (BA1) und 1 bis 90 Gew.-%, mehr bevorzugt 2 bis 75 Gew.-% des Hilfsstoffs (H) bezogen auf das Gesamtgewicht der pharmazeutischen Zubereitung.

Eine weitere Ausführungsform der pharmazeutischen Zubereitung (PZ) betrifft eine halbfeste pharmazeutische Zubereitung (PZ), die das Kohlensäure-Addukt (KA) umfasst, und kutan appliziert wird. Die pharmazeutische Zubereitung (PZ) in dieser Ausführungsform kann beispielsweise in Salbenform, als Creme, als Gel, als Paste, Umschlagspaste oder wirkstoffhaltiges Pflaster appliziert werden.

Vorzugsweise gelten auch für diese Ausführungsform die vorstehend gemachten allgemeinen Angaben zur pharmazeutischen Zubereitung (PZ), insbesondere auch für die Herstellung der pharmazeutischen Zubereitung (PZ), soweit für diese Ausführungsform technisch anwendbar.

Die pharmazeutische Zubereitung (PZ) umfasst in dieser Ausführungsform vorzugsweise mindestens ein Hilfsstoff (H1) ausgewählt aus Paraffinen, insbesondere dick- und dünnflüssigen Paraffinen, Wollwachs, Wollwachsalkoholen, hydrophobes Basisgel, pflanzliche Öle, tierische Fette, synthetische Glyceride, flüssige Polyalkylsiloxane, Wachse, Vaseline und Stärke, insbesondere Maisstärke, vorzugsweise Vaseline.

Unter dickflüssigen Paraffinen *(Paraffinum subliquidum)* werden Paraffine verstanden, die eine Viskosität von 110 bis 230 mPas aufweisen, während dünnflüssige Paraffine (*Paraffinum perliquidum*) eine Viskosität von 25 bis 80 mPas aufweisen.

Vorzugsweise umfasst die pharmazeutische Zubereitung (PZ) in dieser Ausführungsform:
a) 0,1 bis 40 Gew.-%, vorzugsweise 0,4 bis 10 Gew.-% des Kohlensäure-Addukts (KA) und
b) 60 bis 99,9 Gew. -%, vorzugsweise 80 bis 96 Gew.-% des Hilfsstoffs (H1) bezogen auf die Gesamtmenge der pharmazeutischen Zubereitung (PZ) umfasst.

Eine weitere Ausführungsform der pharmazeutischen Zubereitung (PZ), die das Kohlensäure-Addukt (KA) umfasst, betrifft eine pharmazeutische Zubereitung, die parenteral, nasal und/oder inhalativ appliziert wird.

Vorzugsweise gelten auch für diese Ausführungsform die vorstehend gemachten allgemeinen Angaben zur pharmazeutischen Zubereitung (PZ), insbesondere auch für die Zubereitung der pharmazeutischen Zubereitung (PZ), soweit für diese Ausführungsform technisch anwendbar.

Vorzugsweise liegt die pharmazeutische Zubereitung (PZ) in dieser Ausführungsform als Lösung (A2) vor, umfassend das Kohlensäure-Addukt (KA), gelöstes CO₂ und mindestens ein Hilfsstoff (H2).

Der Hilfsstoff (H2) ist vorzugsweise ausgewählt aus einem Alkalihalogenid oder Erdalkalihalogenid, mehr bevorzugt NaCl und MgCl₂, noch mehr bevorzugt NaCl. Der Hilfsstoff (H2) kann identisch mit dem Salz (S) sein. Mengenangaben bezogen auf den Hilfsstoff (H2), soweit dieser in einzelnen Ausführungsformen identisch mit dem Salz (S) ist, beziehen sich im Rahmen dieser Erfindung auf zusätzliche Mengen des Hilfsstoffes (H2), die nicht in Form des Salzes (S) als Teil des Kohlensäure-Adduktes (KA) in die pharmazeutische Zubereitung (PZ) eingebracht wurden.

Vorzugsweise wird die Lösung (A2) durch Einbringen von CO₂ in ein Lösungsmittel erhalten. Vorzugsweise ist das Lösungsmittel Wasser. Vorzugsweise wird das CO₂ zur Herstellung der Lösung (A2) bei einer Temperatur von 0 bis 8 °C, mehr bevorzugt bei 0 bis 5 °C in das Lösungsmittel eingebracht. Das CO₂ kann in Form des Gases als auch in fester Form, etwa als Trockeneis in das Lösungsmittel eingebracht werden. Vorzugsweise wird das CO₂ in Form des Gases in das Lösungsmittel eingebracht. Das CO₂ kann auch unter Druck, bis zum Erreichen der gewünschten Konzentration in die Lösung eingebracht werden, wie vorstehend etwa für Teilschritt a2) beschrieben.

Vorzugsweise wird das CO₂ zur Herstellung der Lösung (A2), bis zu einer Konzentration von mindestens 3 g/l, mehr bevorzugt bis 4 g/l, noch mehr bevorzugt 4 g/l bis 8 g/l in das Lösungsmittel eingebracht.

Vorzugsweise weist das CO₂, dass zur Herstellung der Lösung (A2) verwendet wird mindestens eine Reinheit von 99,9 %, mehr bevorzugt auch eine Qualität auf die für pharmazeutische Anwendungen geeignet ist, wie vorstehend definiert.

Vorzugsweise umfasst die pharmazeutische Zubereitung (PZ) in dieser Ausführungsform, insofern sie durch Auflösen des Kohlensäure-Adduktes (KA) in der Lösung (A2) erhalten wird:
a) 0,05 bis 100 mg/ml, mehr bevorzugt 0,08 bis 50 mg/ml des Kohlensäure-Adduktes (KA) und
b) 0 bis 20 mg/ml, mehr bevorzugt 3 bis 10 mg/ml des Hilfsstoffs (H2)
   bezogen jeweils auf das Gesamtvolumen der pharmazeutischen Zubereitung (PZ).

### BEISPIELE DER ERFINDUNG

### 1. Ausführungsbeispiel 1, Herstellung des Kohlensäure-Adduktes (KA)

### 1.1 Materialien:

- Amin (AM): 68,8 bis 110,1 g Procain-Hydrochlorid (z.B. reinst, zur Verwendung als pharmazeutischer Wirkstoff; Ph. Eur. oder in einer Qualität, die für diesen Zweck geeignet ist)
- Base (BA): 22,2 bis 33,9 g Natriumhydrogencarbonat (z.B. reinst oder in einer Qualität, die für diesen Zweck geeignet ist),
- Lösungsmittel: 630 bis 900 ml Wasser (*Aqua ad iniectabilia*)
- CO₂: mind. 12,0 g/l Kohlendioxid aus Druckgasstahlflaschen (CO₂ in geeigneter Qualität)
- Trockeneis zur Bereitung von Kältemischungen und zur Kühlung
- Methanol, techn. zur Bereitung von Kältemischungen

### 1.2 Schritt a)

In eine gereinigte Kunststoffdruckflasche wird Wasser (z.B. *Aqua ad iniectabilia*) bis zur Markierung eingefüllt (ca. 800 bis 900 ml) und für mindestens 1 h im Kühlschrank (3 bis 8 °C) oder mittels externer Kühlung auf 5 °C vorgekühlt.

Es wird eine mit Kohlendioxid gesättigte Kohlensäurelösung bereitet. Dafür wird CO₂ intervallweise unter Druck (1,6 bis 8 bar) in das vorgekühlte Wasser eingebracht. Das Zischen (entweichendes Gas über das Überdruckventil) zeigt Sättigung der Lösung mit CO₂ an. Die Sättigung wird über das Gewicht kontrolliert, bis 4,0 bis 6,0 g CO₂ (entsprechend 4,5 bis 7,5 g/l) gelöst sind. Die gesättigte Lösung weist einen pH-Wert von ≤ 4,3 bis 4,8 auf. Dieses kohlensäurehaltige Wasser wird unmittelbar verschlossen sowie mindestens für 1 h im Kühlschrank aufbewahrt.

### 1.3 Schritt b)

In einer zweiten Kunststoffdruckflasche wird 21,2 g Natriumhydrogencarbonat vorgelegt, mit 320 ml gekühltem kohlensäurehaltigen Wasser versetzt und unter Schwenken aufgelöst.

### 1.4 Schritt c)

Zu dieser Lösung wird die äquivalente Menge an festem Procain-Hydrochlorid bei gleichbleibender Temperatur gegeben, wobei sich eine nahezu neutrale Lösung bildet, die nach Zugabe von weiteren 320 ml kalten kohlensäurehaltigen Wasser eine klare schwach saure Lösung ergibt. Die Lösung wird mit CO₂ angereichert. Die so bereitete Lösung wird für mindestens eine 1 h im Kühlschrank gelagert.

Anschließend wird die Lösung nochmals mit CO₂ konditioniert, bis eine CO₂-Konzentration von 12 g/l in der Lösung erreicht ist Mittels pH-Indikatorstäbchen wird der pH-Wert kontrolliert. Der pH-Wert beträgt ≤ 6,6.

### 1.5 Schritt d)

Rundkolben werden vorgekühlt. Zum Einfrieren wird die Reaktionslösung in einem vorgekühlten Messzylinder abgemessen, portionsweise in Rundkolben überführt und durch Eintauchen in eine Trockeneis-/Methanol-Kältemischung (<-60°C) nach dem Shell-freezing-Verfahren innerhalb von 1,5 bis 3,5 min pro Kolben eingefroren (-200 rpm). Der Eintauchwinkel des Kolbens am Rotationsverdampfer wird auf ca. 40 ° eingestellt.

### 1.6 Schritt e)

Die Kolben mit dem so eingefrorenen Gut werden mit einem Schliffstopfen verschlossen und in einem Tiefkühlschrank bei -15 bis -20 °C für 2 bis 4 Tage zwischengelagert.

### 1.7 Schritt f)

Die so temperierten Kolben werden mit Styroporbehältern ummantelt, die vorgekühlt sind und unverzüglich einzeln an eine evakuierte (0,060 ± 0,01 mbar, ca. -46 °C, Dichtheitsprobe) Gefriertrocknungsanlage, über einen flexiblen Gummikegel angeschlossen. Die Ventilhähne werden vorsichtig geöffnet und die einzelnen Kolben unter Vakuum gesetzt. Abschließend müssen alle Kolben evakuiert sein.

Zur Überwachung des Prozesses werden unten im Styropormantel Temperaturfühler platziert, die während der gesamten Trocknung den gesamten Temperaturverlauf aufzeichnen. Vor dem Start der Lyophilisation zeigen die Temperaturfühler Temperaturen von < -5 °C an.

Während der Lyophilisation beträgt der Druck 0,07 ± 0,02 mbar. Dieser Sublimationsdruck wird innerhalb von 4 h erreicht und während der gesamten Lyophilisationszeit beibehalten. Die Kühlkammer wird während der gesamten Trocknung auf 9 bis 15 °C temperiert. Der Endpunkt der Lyophilisation wird graphisch aus den Temperaturverlaufsaufzeichnungen ermittelt. Die Gesamttrocknungszeit betrug maximal 52 h. Das trockene Lyophilisat wird in ein Braunglasgefäß mit Twist-off-Deckel überführt, mit einem Trockenmittelbeutel versehen und im Kühlschrank bei 0 bis 15 °C gelagert.

### 2. Beispiele für Pharmazeutische Zubereitungen (PZ)

### 2.1 Kapseln und Tabletten

Nachfolgend wird beispielhaft die Zusammensetzung der erfindungsgemäßen pharmazeutischen Zubereitung (PZ) in der Ausführungsform als Kapsel oder Tablette für Procain als Amin (AM) beschrieben. Für die Herstellung der Kapseln können handelsübliche Steckkapseln in den handelsüblichen Größen (5 bis 000) verwendet werden, die mit dem Kohlensäure-Addukt (KA) haltigen Pulver, umfassend Procain als Amin (AM) (Verreibung des Wirkstoffs Kohlensäure-Addukt (KA), umfassend Procain als Amin (AM) ggf. mit Zusätzen, Füllstoffen, und Fließregulierungsmittel) befüllt werden. Das Kohlensäure-Addukt (KA) wurde gemäß Beispiel 1 hergestellt. Es hat sich gezeigt, dass Hartgelatine-Kapseln gegenüber Cellulose-Kapseln im Hinblick auf die Stabilität besser geeignet sind. So wiesen die befüllten Hartgelatinekapseln, beispielhaft für Hartgelatinekapseln mit 60 und 100 mg Wirkstoff gemäß Tabelle 1, auch nach 12 Monaten Lagerung im Kühlschrank keine Veränderungen auf und sind somit stabil. Die Stabilität wurde mittels IR-Spektroskopie untersucht. So wurde im Fall der Hartgelatine-Kapseln innerhalb des 12 Monatszeitraumes IR-spektroskopisch kein Procain detektiert, während für Cellulose- Kapseln bereits nach wenigen Tagen eine Procain-Bande im IR-Spektrum gemessen wurde. Die Gehaltsbestimmung erfolgte bei Raumtemperatur mit UV/VIS-Spektroskopie. Für Rezepturarzneimittel wird gemäß *Pharmacopoea Europaea* Punkt 2.9.6 bezogen auf den Gesamtgehalt inklusive Nebenprodukte ein Toleranzbereich von ±15 % vorgeschrieben.
Es werden die geltenden und als allgemein üblichen pharmazeutischen Regeln zur Herstellung von (Rezeptur)-Arzneimitteln angewendet (zum Beispiel *Pharmacopoea Europaea,* Deutscher Arzneimittel-Codex).

**Tabelle 1: Beispielzusammensetzung Kapseln mit NaHCO₃-Zusatz und Kohlensäure-Addukt (KA) als Wirkstoff hergestellt gemäß Ausführungsbeispiel 1.**

| | | | | |
|---|---|---|---|---|
| Wirkstoffmenge [mg] | 30 | 60 | 100 | 120 |
| Zusatz, z.B. NaHCO₃ | 21 | 42 | 71 | 84 |
| Füllstoff, z.B. Maisstärke inkl. SiO₂ | 120 | 90 | 120 | 90 |
| Kapselgröße | 1 | 1 | 0 | 0 |

**Tabelle 2: Beispielzusammensetzung Tablette mit NaHCO₃-Zusatz und Kohlensäure-Addukt (KA) als Wirkstoff hergestellt gemäß Ausführungsbeispiel 1.**

| | | | | |
|---|---|---|---|---|
| Wirkstoffmenge [mg] | 30 | 60 | 100 | 120 |
| Zusatz, z.B. NaHCO₃ | 21 | 42 | 71 | 84 |
| Füllstoff, z.B. Maisstärke inkl. SiO₂ | 2,5 | 5 | 8,5 | 10 |

### 2.2 Salben

Nachfolgend wird die Zusammensetzung der erfindungsgemäßen pharmazeutischen Zubereitung (PZ) in der Ausführungsform als Salbe beispielhaft für Procain als Amin (AM) im Kohlensäure-Addukt (KA) erläutert. Bei der Bereitung der Salbe werden die geltenden und allgemein üblichen pharmazeutischen Regeln zur Herstellung von (Rezeptur)-Arzneimitteln angewendet (zum Beispiel *Pharmacopoea Europaea,* Deutscher Azneimittel-Codex). Bei der Herstellung der Salbe werden größere Scherkräfte vermieden. Außerdem wird die Temperatur bei der Zubereitung auch lokal unter 60 °C gehalten. So wird das gemörserte Kohlensäure-Addukt (KA) umfassend Procain als Amin (AM) in einem Mörser oder einer Fantaschale, die mittels eines auf 40 bis 45 °C temperierten Wasserbades, temperiert werden, in die Salbengrundlage, beispielsweise Vaseline, eingebracht. Alternativ ist auch die Verwendung von elektrischen Mischsystemen möglich, wie sie im Rahmen des üblichen Apothekenbetriebes zum Einsatz kommen.

**Tabelle 3: Beispielzusammensetzung Salbe und Kohlensäure-Addukt (KA) als Wirkstoff hergestellt gemäß Ausführungsbeispiel 1.**

| | | | | | |
|---|---|---|---|---|---|
| Gehalt [%] | 0,5 | 1,0 | 1,25 | 2 | 4 |
| Wirkstoffmenge [mg] | 25 | 50 | 62,5 | 100 | 200 |
| Salbengrundlage [g] (z.B. Vaseline) | 5 | 5 | 5 | 5 | 5 |

### 2.3 Parenterale Lösungen

Für die Bereitung einer parenteralen Lösung die das Kohlensäure-Addukt (KA) umfassend Procain als Amin (AM) enthält, wird in einem geeigneten Gefäß mit einem Rührfisch oder Ähnlichem die erforderliche Menge Wasser (*Aqua ad iniectabilia*) auf ca. 5 ±3 °C gekühlt und bei dieser Temperatur belassen. Das Wasser wird mit gasförmigen Kohlendioxid in der benötigten Qualität auf ca. 3,2 g/l angereichert. In diesem kohlensäurehaltigen Wasser wird die entsprechende Menge Kohlensäure-Addukt (KA) umfassend Procain als Amin (AM) und Natriumchlorid für einen isotonischen Gehalt gelöst.

Alternativ wird auf ca. 5 ± 3 °C temperiertes Wasser unter Druck in einem geschlossenen System mit CO₂ so angereichert, dass ein deutlicher Überschuss vorhanden ist (4,5 bis 7,5 g/l). Zu diesem kohlensäurehaltigen Wasser werden ebenfalls die entsprechenden Mengen Kohlensäure-Addukt (KA) umfassend Procain als Amin (AM) und Natriumchlorid gegeben.

Diese kalte mit dem Kohlensäure-Addukt (KA) umfassend Procain als Amin (AM) und Natriumchlorid versehene Lösung wird unter geeigneten räumlichen Bedingungen sterilfiltriert und in entsprechende Vials abgefüllt. Es werden die geltenden und allgemein üblichen pharmazeutischen Regeln zur Herstellung von (Rezeptur)-Arzneimitteln angewendet (zum Beispiel *Pharmacopoea Europaea*)*.*

**Tabelle 4: Beispielzusammensetzungen für parenterale Zubereitungen mit NaCl-Zusatz und Kohlensäure-Addukt (KA) als Wirkstoff hergestellt gemäß Ausführungsbeispiel 1.**

| | Infusion | | | Injektion | | |
|---|---|---|---|---|---|---|
| Gehalt [%] | 0,1 | 0,2 | 0,3 | 1 | 2 | 3 |
| Wirkstoffmenge [mg] | 50 | 100 | 150 | 50 | 100 | 150 |
| Zusatz (NaCl) [mg] | 440 | 430 | 425 | 37 | 29 | 25 |
| Volumen gesamt [ml] | 50 | 50 | 50 | 5 | 5 | 5 |

### 3. Löslichkeit in Oktanol

Es wurden Löslichkeitsversuche in Oktanol durchgeführt und der Gehalt in der organischen Phase UV/VIS-spektroskopisch sowie die pH-Werte untersucht.

**Tabelle 5: Löslichkeit verschiedener Darreichungsformen**

| **Substanz** | **Gehalt** | **pH-Wert** |
|---|---|---|
| Procain | komplett gelöst (100 %) | 8,5 |
| Procain-Hydrochlorid (Proc HCl) | 8% | 4,5 |
| Kohlensäure-Addukt des Procains | 74% | 7,5 |

Die ermittelten pH-Werte belegen, dass das Kohlensäure-Addukt des Procains als solches und nicht in Procain überführt und in dieser Form in der organischen Phase löslich und somit membrangängig ist. Ebenso ist anhand der UV/VIS-spektroskopischen Gehaltsmessung erkennbar, dass das Kohlensäure-Addukt des Procains in puncto Lipophilie dem Procain ähnlicher ist als dem Procain HCl. Es verhält sich also wie die basische Komponente - die lipidlösliche Base, die in Abhängigkeit vom pH-Wert gebildet wird. Das Kohlensäure-Addukt des Procains weist diese Eigenschaft unabhängig vom pH-Wert auf, d.h. es muss nicht wie beim Procain HCl durch eine pH-Wert-Änderung in die lipophile Form überführt werden. Procain HCl weist einen deutlich niedrigeren Wert auf, der mit 8 % in der Größenordnung für die Eiweißbindung von 6 % liegt.

### LITERATUR

1. Clayville LR. 2011. Influenza update: a review of currently available vaccines. P T 36:659-84.
2. Taubenberger JK, Kash JC, Morens DM. 2019. The 1918 influenza pandemic: 100 years of questions answered and unanswered. Sci Transl Med 11.
3. Morens DM, Taubenberger JK, Fauci AS. 2008. Predominant role of bacterial pneumonia as a cause of death in pandemic influenza: implications for pandemic influenza preparedness. J Infect Dis 198:962-70.
4. McCullers JA. 2014. The co-pathogenesis of influenza viruses with bacteria in the lung. Nat Rev Microbiol 12:252-62.
5. Vanderbeke L, Spriet I, Breynaert C, Rijnders BJA, Verweij PE, Wauters J. 2018. Invasive pulmonary aspergillosis complicating severe influenza: epidemiology, diagnosis and treatment. Curr Opin Infect Dis 31:471-480.
6. Nulens EF, Bourgeois MJ, Reynders MB. 2017. Post-influenza aspergillosis, do not underestimate influenza B. Infect Drug Resist 10:61-67.
7. Alobaid K, Alfoudri H, Jeragh A. 2020. Influenza-associated pulmonary aspergillosis in a patient on ECMO. Med Mycol Case Rep 27:36-38.
8. Huang L, Zhang N, Huang X, Xiong S, Feng Y, Zhang Y, Li M, Zhan Q. 2019. Invasive pulmonary aspergillosis in patients with influenza infection: A retrospective study and review of the literature. Clin Respir J 13:202-211.
9. Verweij PE, Rijnders BJA, Bruggemann RJM, Azoulay E, Bassetti M, Blot S, Calandra T, Clancy CJ, Cornely OA, Chiller T, Depuydt P, Giacobbe DR, Janssen NAF, Kullberg BJ, Lagrou K, Lass-Florl C, Lewis RE, Liu PW, Lortholary O, Maertens J, Martin-Loeches I, Nguyen MH, Patterson TF, Rogers TR, Schouten JA, Spriet I, Vanderbeke L, Wauters J, van de Veerdonk FL. 2020. Review of influenza-associated pulmonary aspergillosis in ICU patients and proposal for a case definition: an expert opinion. Intensive Care Med 46: 1524-1535.
10. Ostrosky-Zeichner L, Casadevall A, Galgiani JN, Odds FC, Rex JH. 2010. An insight into the antifungal pipeline: selected new molecules and beyond. Nat Rev Drug Discov 9:719-27.
11. Tobin JM, Nickolich KL, Ramanan K, Pilewski MJ, Lamens KD, Alcorn JF, Robinson KM. 2020. Influenza Suppresses Neutrophil Recruitment to the Lung and Exacerbates Secondary Invasive Pulmonary Aspergillosis. J Immunol 205:480-488.
12. Wiederhold NP, Verweij PE. 2020. Aspergillus fumigatus and pan-azole resistance: who should be concerned? Curr Opin Infect Dis 33:290-297.
13. Duwe SC, Schmidt B, Gartner BC, Timm J, Adams O, Fickenscher H, Schmidtke M. 2021. Prophylaxis and treatment of influenza: options, antiviral susceptibility, and existing recommendations. GMS Infect Dis 9:Doc02.
14. Hurt AC, Besselaar TG, Daniels RS, Ermetal B, Fry A, Gubareva L, Huang W, Lackenby A, Lee RT, Lo J, Maurer-Stroh S, Nguyen HT, Pereyaslov D, Rebelo-de-Andrade H, Siqueira MM, Takashita E, Tashiro M, Tilmanis D, Wang D, Zhang W, Meijer A. 2016. Global update on the susceptibility of human influenza viruses to neuraminidase inhibitors, 2014-2015. Antiviral Res 132:178-85.
15. Ludwig S. 2011. Disruption of virus-host cell interactions and cell signaling pathways as an anti-viral approach against influenza virus infections. Biol Chem 392:837-47.
16. Lampejo T. 2020. Influenza and antiviral resistance: an overview. Eur J Clin Microbiol Infect Dis 39:1201-1208.
17. Häring C, Schroeder J, Löffler B, Engert B, Ehrhardt C. 2021. The local anaesthetic procaine prodrugs ProcCluster<sup>®</sup> and Procaine-hydrochloride impair SARS-CoV-2 replication doi:10.1101/2021.06.07.447335:2021.06.07.447335.
18. Abdelaziz AA, el-Nakeeb MA. 1987. Sporicidal activity of local anaesthetics and their binary combinations with preservatives. J Basic Microbiol 27:403-10.
19. Rodrigues AG, Araujo R, Pina-Vaz C. 2006. Interaction of local anaesthetics with other antifungal agents against pathogenic Aspergillus. Int J Antimicrob Agents 27:339-43.
20. Krammer F, Smith GJD, Fouchier RAM, Peiris M, Kedzierska K, Doherty PC, Palese P, Shaw ML, Treanor J, Webster RG, Garcia-Sastre A. 2018. Influenza. Nat Rev Dis Primers 4:3.
21. Kim H, Webster RG, Webby RJ. 2018. Influenza Virus: Dealing with a Drifting and Shifting Pathogen. Viral Immunol 31:174-183.
22. Morens DM, Taubenberger JK. 2018. The Mother of All Pandemics Is 100 Years Old (and Going Strong)! Am J Public Health 108:1449-1454.
23. Taubenberger JK, Reid AH, Lourens RM, Wang R, Jin G, Fanning TG. 2005. Characterization of the 1918 influenza virus polymerase genes. Nature 437:889-93.
24. Morris DE, Cleary DW, Clarke SC. 2017. Secondary Bacterial Infections Associated with Influenza Pandemics. Front Microbiol 8:1041.
25. Podewils LJ, Liedtke LA, McDonald LC, Hageman JC, Strausbaugh LJ, Fischer TK, Jernigan DB, Uyeki TM, Kuehnert MJ, Infectious Diseases Society of America Emerging Infections N. 2005. A national survey of severe influenza-associated complications among children and adults, 2003-2004. Clin Infect Dis 40:1693-6.
26. Iverson AR, Boyd KL, McAuley JL, Plano LR, Hart ME, McCullers JA. 2011. Influenza virus primes mice for pneumonia from Staphylococcus aureus. J Infect Dis 203:880-8.
27. Ku YH, Chan KS, Yang CC, Tan CK, Chuang YC, Yu WL. 2017. Higher mortality of severe influenza patients with probable aspergillosis than those with and without other coinfections. J Formos Med Assoc 116:660-670.
28. Kosmidis C, Denning DW. 2015. The clinical spectrum of pulmonary aspergillosis. Thorax 70:270-7.
29. van de Veerdonk FL, Gresnigt MS, Romani L, Netea MG, Latge JP. 2017. Aspergillus fumigatus morphology and dynamic host interactions. Nat Rev Microbiol 15:661-674.
30. Duan Y, Ou X, Chen Y, Liang B, Ou X. 2020. Severe Influenza With Invasive Pulmonary Aspergillosis in Immunocompetent Hosts: A Retrospective Cohort Study. Front Med (Lausanne) 7:602732.
31. Shah MM, Hsiao El, Kirsch CM, Gohil A, Narasimhan S, Stevens DA. 2018. Invasive pulmonary aspergillosis and influenza co-infection in immunocompetent hosts: case reports and review of the literature. Diagn Microbiol Infect Dis 91:147-152.
32. Ludwig S. 2009. Targeting cell signalling pathways to fight the flu: towards a paradigm change in anti-influenza therapy. J Antimicrob Chemother 64:1-4.
33. Meineke R, Rimmelzwaan GF, Elbahesh H. 2019. Influenza Virus Infections and Cellular Kinases. Viruses 11.
34. Haasbach E, Muller C, Ehrhardt C, Schreiber A, Pleschka S, Ludwig S, Planz O. 2017. The MEK-inhibitor CI-1040 displays a broad anti-influenza virus activity in vitro and provides a prolonged treatment window compared to standard of care in vivo. Antiviral Res 142:178-184.
35. Holzberg M, Boergeling Y, Schrader T, Ludwig S, Ehrhardt C. 2017. Vemurafenib Limits Influenza A Virus Propagation by Targeting Multiple Signaling Pathways. Front Microbiol 8:2426.
36. Ludwig S, Wolff T, Ehrhardt C, Wurzer WJ, Reinhardt J, Planz O, Pleschka S. 2004. MEK inhibition impairs influenza B virus propagation without emergence of resistant variants. FEBS Lett 561:37-43.
37. Marjuki H, Alam MI, Ehrhardt C, Wagner R, Planz O, Klenk HD, Ludwig S, Pleschka S. 2006. Membrane accumulation of influenza A virus hemagglutinin triggers nuclear export of the viral genome via protein kinase Calpha-mediated activation of ERK signaling. J Biol Chem 281:16707-15.
38. Pleschka S, Wolff T, Ehrhardt C, Hobom G, Planz O, Rapp UR, Ludwig S. 2001. Influenza virus propagation is impaired by inhibition of the Raf/MEK/ERK signalling cascade. Nat Cell Biol 3:301-5.
39. Schrader T, Dudek SE, Schreiber A, Ehrhardt C, Planz O, Ludwig S. 2018. The clinically approved MEK inhibitor Trametinib efficiently blocks influenza A virus propagation and cytokine expression. Antiviral Res 157:80-92.
40. Ehrhardt C, Ruckle A, Hrincius ER, Haasbach E, Anhlan D, Ahmann K, Banning C, Reiling SJ, Kuhn J, Strobl S, Vitt D, Leban J, Planz O, Ludwig S. 2013. The NF-kappaB inhibitor SC75741 efficiently blocks influenza virus propagation and confers a high barrier for development of viral resistance. Cell Microbiol 15:1198-211.
41. Haasbach E, Reiling SJ, Ehrhardt C, Droebner K, Ruckle A, Hrincius ER, Leban J, Strobl S, Vitt D, Ludwig S, Planz O. 2013. The NF-kappaB inhibitor SC75741 protects mice against highly pathogenic avian influenza A virus. Antiviral Res 99:336-44.
42. Mazur I, Wurzer WJ, Ehrhardt C, Pleschka S, Puthavathana P, Silberzahn T, Wolff T, Planz O, Ludwig S. 2007. Acetylsalicylic acid (ASA) blocks influenza virus propagation via its NF-kappaB-inhibiting activity. Cell Microbiol 9:1683-94.
43. Deinhardt-Emmer S, Jackel L, Haring C, Bottcher S, Wilden JJ, Gluck B, Heller R, Schmidtke M, Koch M, Loffler B, Ludwig S, Ehrhardt C. 2021. Inhibition of Phosphatidylinositol 3-Kinase by Pictilisib Blocks Influenza Virus Propagation in Cells and in Lungs of Infected Mice. Biomolecules 11.
44. Ehrhardt C, Ludwig S. 2009. A new player in a deadly game: influenza viruses and the Pl3K/Akt signalling pathway. Cell Microbiol 11:863-71.
45. Ehrhardt C, Marjuki H, Wolff T, Nurnberg B, Planz O, Pleschka S, Ludwig S. 2006. Bivalent role of the phosphatidylinositol-3-kinase (Pl3K) during influenza virus infection and host cell defence. Cell Microbiol 8:1336-48.
46. Ehrhardt C, Wolff T, Ludwig S. 2007. Activation of phosphatidylinositol 3-kinase signaling by the nonstructural NS1 protein is not conserved among type A and B influenza viruses. J Virol 81:12097-100.
47. Ehrhardt C, Wolff T, Pleschka S, Planz O, Beermann W, Bode JG, Schmolke M, Ludwig S. 2007. Influenza A virus NS1 protein activates the Pl3K/Akt pathway to mediate antiapoptotic signaling responses. J Virol 81:3058-67.
48. Eierhoff T, Hrincius ER, Rescher U, Ludwig S, Ehrhardt C. 2010. The epidermal growth factor receptor (EGFR) promotes uptake of influenza A viruses (IAV) into host cells. PLoS Pathog 6:e1001099.
49. Day AM, Quinn J. 2019. Stress-Activated Protein Kinases in Human Fungal Pathogens. Front Cell Infect Microbiol 9:261.
50. Song J, Pan W, Sun Y, Han J, Shi W, Liao W. 2017. Aspergillus fumigatus-induced early inflammatory response in pulmonary microvascular endothelial cells: Role of p38 MAPK and inhibition by silibinin. Int Immunopharmacol 49:195-202.
51. Denning DW, Hope WW. 2010. Therapy for fungal diseases: opportunities and priorities. Trends Microbiol 18:195-204.
52. Ji Y, Yang F, Ma D, Zhang J, Wan Z, Liu W, Li R. 2012. HOG-MAPK signaling regulates the adaptive responses of Aspergillus fumigatus to thermal stress and other related stress. Mycopathologia 174:273-82.
53. Hagiwara D, Suzuki S, Kamei K, Gonoi T, Kawamoto S. 2014. The role of AtfA and HOG MAPK pathway in stress tolerance in conidia of Aspergillus fumigatus. Fungal Genet Biol 73: 138-49.
54. Yang G, Cao X, Ma G, Qin L, Wu Y, Lin J, Ye P, Yuan J, Wang S. 2020. MAPK pathway-related tyrosine phosphatases regulate development, secondary metabolism and pathogenicity in fungus Aspergillus flavus. Environ Microbiol 22:5232-5247.
55. Manfiolli AO, Dos Reis TF, de Assis LJ, de Castro PA, Silva LP, Hori Jl, Walker LA, Munro CA, Rajendran R, Ramage G, Goldman GH. 2018. Mitogen activated protein kinases (MAPK) and protein phosphatases are involved in Aspergillus fumigatus adhesion and biofilm formation. Cell Surf 1:43-56.
56. Schloer S, Goretzko J, Kuhnl A, Brunotte L, Ludwig S, Rescher U. 2019. The clinically licensed antifungal drug itraconazole inhibits influenza virus in vitro and in vivo. Emerg Microbes Infect 8:80-93.
57. Schloer S, Goretzko J, Pleschka S, Ludwig S, Rescher U. 2020. Combinatory Treatment with Oseltamivir and Itraconazole Targeting Both Virus and Host Factors in Influenza A Virus Infection. Viruses 12.
58. Schloer S, Brunotte L, Mecate-Zambrano A, Zheng S, Tang J, Ludwig S, Rescher U. 2021. Drug synergy of combinatory treatment with remdesivir and the repurposed drugs fluoxetine and itraconazole effectively impairs SARS-CoV-2 infection in vitro. Br J Pharmacol 178:2339-2350.
59. Borgeling Y, Schmolke M, Viemann D, Nordhoff C, Roth J, Ludwig S. 2014. Inhibition of p38 mitogen-activated protein kinase impairs influenza virus-induced primary and secondary host gene responses and protects mice from lethal H5N1 infection. J Biol Chem 289:13-27.
60. Yu J, Sun X, Goie JYG, Zhang Y. 2020. Regulation of Host Immune Responses against Influenza A Virus Infection by Mitogen-Activated Protein Kinases (MAPKs). Microorganisms 8.
61. Scheuch G, Canisius S, Nocker K, Hofmann T, Naumann R, Pleschka S, Ludwig S, Welte T, Planz O. 2018. Targeting intracellular signaling as an antiviral strategy: aerosolized LASAG for the treatment of influenza in hospitalized patients. Emerg Microbes Infect 7:21.

## Patentansprüche

1. Ein Amin (AM) gemäß der allgemeinen Formel (I) 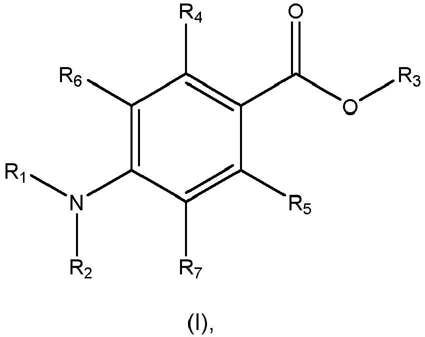 wobei in Formel (I)
R₁ ist H, (C₁₋₁₀)Alkyl, (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₂ ist H, (C₁₋₁₀Alkyl), (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₃ ist -(CH₂)ₙNR₈R₉;
n ist 1 bis 5, vorzugsweise 1 bis 3, mehr bevorzugt 1 bis 2,
R₈ ist (C₁₋₁₀)Alkyl, vorzugsweise (C₁₋₂)Alkyl,
R₉ ist (C₁₋₁₀)Alkyl, vorzugsweise (C₁₋₂)Alkyl;
R₄ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀)Alkyl, vorzugsweise H, Halogen, (C₁₋₁₀)Alkyl oder -O(C₁₋₁₀) Alkyl, mehr bevorzugt H oder Halogen;
R₅ ist H, Halogen, (C₁₋₁₀)Alkyl, (C₂₋₁₀)Alkenyl, (C₅-C₁₄)Aryl, (C₅-C₁₀)Heteroaryl oder -O-(C₁₋₁₀)Alkyl, vorzugsweise H, Halogen, (C₁₋₁₀)Alkyl oder-O(C₁₋₁₀)Alkyl, mehr bevorzugt H oder Halogen;
R₆ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder-O(C₁₋₁₀)Alkyl, mehr bevorzugt H oder -O(C₁₋₁₀)Alkyl;
R₇ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder-O(C₁₋₁₀)Alkyl, mehr bevorzugt H oder-O-(C₁₋₁₀)Alkyl; wobei das Amin gemäß Formel (I) optional auch in Form eines Salzes eingesetzt werden kann, zur Verwendung in der Behandlung von Infektionen mit *Aspergillus fumigatus* und von Superinfektionen mit Influenzaviren und *Aspergillus spp..*

2. Ein Kohlensäure-Addukt (KA) umfassend mindestens ein Strukturelement gemäß der allgemeinen Formel (II), (III) und/oder (IV) 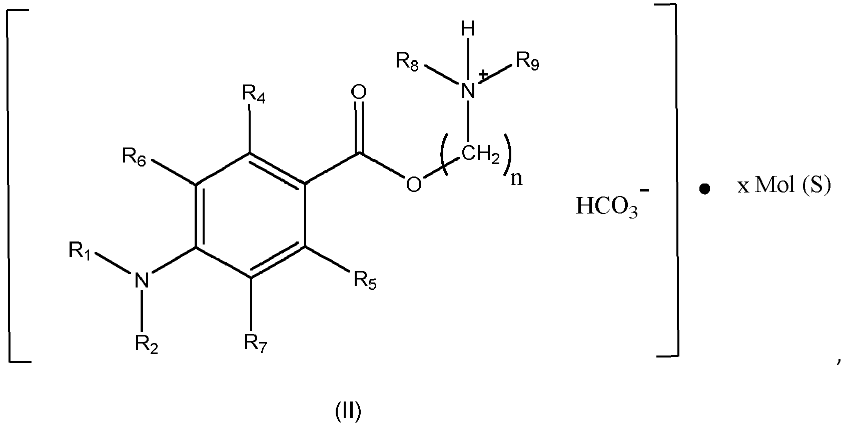 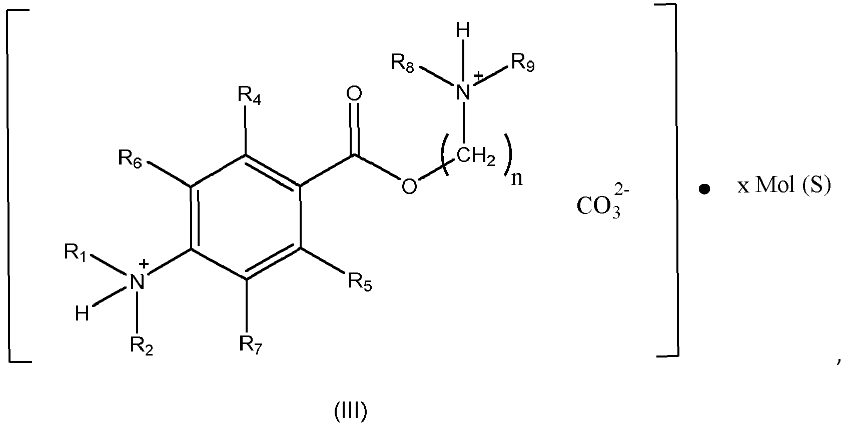 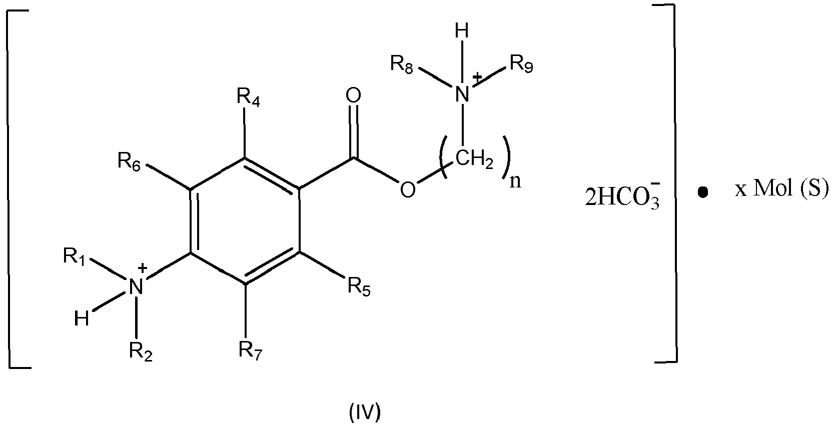 wobei in Formel (II), (III), und (IV)
R₁ ist H, (C₁₋₁₀)Alkyl, (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₂ ist H, (C₁₋₁₀Alkyl), (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₃ ist -(CH₂)ₙNR₈R₉;
n ist 1 bis 5, vorzugsweise 1 bis 3, mehr bevorzugt 1 bis 2,
R₈ ist (C₁₋₁₀)Alkyl, vorzugsweise (C₁₋₂)Alkyl,
R₉ ist (C₁₋₁₀)Alkyl, vorzugsweise (C₁₋₂)Alkyl;
R₄ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀)Alkyl, vorzugsweise H, Halogen, (C₁₋₁₀)Alkyl oder -O(C₁₋₁₀) Alkyl, mehr bevorzugt H oder Halogen;
R₅ ist H, Halogen, (C₁₋₁₀)Alkyl, (C₂₋₁₀)Alkenyl, (C₅-C₁₄)Aryl, (C₅-C₁₀)Heteroaryl oder -O-(C₁₋₁₀)Alkyl, vorzugsweise H, Halogen, (C₁₋₁₀)Alkyl oder-O(C₁₋₁₀)Alkyl, mehr bevorzugt H oder Halogen;
R₆ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder-O(C₁₋₁₀)Alkyl, mehr bevorzugt H oder -O(C₁₋₁₀)Alkyl;
R₇ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder-O(C₁₋₁₀)Alkyl, mehr bevorzugt H oder -O(C₁₋₁₀)Alkyl;
x is 0,5 bis 30;
(S) ist ein Salz.
zur Verwendung in der Behandlung von Infektionen mit *Aspergillus fumigatus* und von Superinfektionen mit Influenzaviren und *Aspergillus spp..*

3. Ein Kohlensäure-Addukt (KA) umfassend Kohlensäure, mindestens ein Amin (AM) gemäß der allgemeinen Formel (I) und mindestens ein Salz (S), 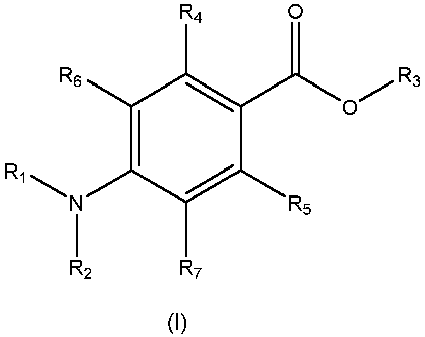 wobei in Formel (I)
R₁ ist H, (C₁₋₁₀)Alkyl, (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₂ ist H, (C₁₋₁₀Alkyl), (C₂₋₁₀)Alkenyl, (C₅-C₁₄)Aryl oder (C₅-C₁₀)Heteroaryl, vorzugsweise H oder (C₁₋₁₀)Alkyl, mehr bevorzugt H;
R₃ ist -(CH₂)ₙNR₈R₉;
n ist 1 bis 5, vorzugsweise 1 bis 3, mehr bevorzugt 1 bis 2,
R₈ ist (C₁₋₁₀)Alkyl, vorzugsweise (C₁₋₂)Alkyl,
R₉ ist (C₁₋₁₀)Alkyl, vorzugsweise (C₁₋₂)Alkyl;
R₄ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀)Alkyl, vorzugsweise H, Halogen, (C₁₋₁₀)Alkyl oder-O(C₁₋₁₀) Alkyl, mehr bevorzugt H oder Halogen;
R₅ ist H, Halogen, (C₁₋₁₀)Alkyl, (C₂₋₁₀)Alkenyl, (C₅-C₁₄)Aryl, (C₅-C₁₀)Heteroaryl oder -O-(C₁₋₁₀)Alkyl, vorzugsweise H, Halogen, (C₁₋₁₀)Alkyl oder-O(C₁₋₁₀)Alkyl, mehr bevorzugt H oder Halogen;
R₆ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder-O(C₁₋₁₀)Alkyl, mehr bevorzugt H oder -O(C₁₋₁₀)Alkyl;
R₇ ist H, Halogen, (C₁₋₁₀)Alkyl, (C₂₋₁₀)Alkenyl, (C₅-C₁₄)Aryl, (C₅-C₁₀)Heteroaryl oder -O(C₁₋₁₀) Alkyl, vorzugsweise H, Halogen, (C₁₋₁₀)Alkyl oder-O(C₁₋₁₀)Alkyl, mehr bevorzugt H oder-O(C₁₋₁₀)Alkyl; wobei das mindestens eine Amin gemäß Formel (I) optional auch in Form eines Salzes eingesetzt werden kann;
herstellbar nach einem Verfahren umfassend die Schritte:
a) Bereitstellung einer Lösung (A), die mindestens ein Lösungsmittel und in dem mindestens einem Lösungsmittel gelöstes CO₂ umfasst,
optional b) Auflösen einer Base (BA), die nicht dem Amin (AM) entspricht, in der Lösung (A) unter Erhalt der Lösung (A1),
c) Lösen des mindestens einen Amins (AM) in der Lösung (A) oder (A1), unter Erhalt der Lösung (B),
d) Einfrieren der nach Abschluss des Schrittes c) erhaltenen Lösung,
e) Lagerung der in Schritt d) eingefrorenen Lösung bei -100 bis 0 °C für nicht länger als 4 Tage;
zur Verwendung in der Behandlung von Infektionen mit *Aspergillus fumigatus* und von Superinfektionen mit Influenzaviren und *Aspergillus spp..*

4. Eine pharmazeutische Zusammensetzung (PZ) umfassend das Amin (AM) gemäß Anspruch 1, oder das Kohlensäure-Addukt gemäß einem der Ansprüche 2 oder 3 zur Verwendung in der Behandlung von Infektionen mit *Aspergillus fumigatus* und von Superinfektionen mit Influenzaviren und *Aspergillus spp..*

5. Das Amin zur Verwendung gemäß Anspruch 1, das Kohlensäure-Addukt (KA) zur Verwendung gemäß einem der Ansprüche 2, oder 3, und die pharmazeutische Zusammensetzung (PZ) zur Verwendung gemäß Anspruch 4, wobei
i) das Amin gemäß der allgemeinen Formel (I) sowie in den allgemeinen Formeln (II), (III) und (IV) ausgewählt ist aus der Gruppe bestehend aus, 4-Aminobenzoesäure-2-(*N*,*N*-diethylamino-)ethylester (Procain), 4-Aminobenzoesäureethylester (Benzocain), 2-(Diethylamino)ethyl-4-amino-2-chlorbenzoat (Chlorprocain), 4-Amino-3-butoxybenzoesäure-2-diethylaminoethylester (Oxybuprocain), (2-(Dimethylamino)ethyl)-4-(butylamino)benzoat (Tetracain), vorzugsweise 4-Aminobenzoesäure-2-(*N*,*N*-diethylamino-)ethylester (Procain) und/oder
ii) das Salz (S) ein Salz ist, das zusammengesetzt ist, aus mindestens einem Kation ausgewählt aus Na⁺, K⁺, Li⁺, Mg²⁺, Zn²⁺, Fe²⁺, Fe³⁺ und Mn²⁺ , vorzugsweise Na⁺ und mindestens einem Anion ausgewählt aus Cl⁻, Br⁻, J⁻, F⁻, SO₄²⁻, SO₃²⁻, HSO₄⁻ HSO₃⁻, ⁻HCO₃⁻, CO₃²⁻, PO₄³⁻, HPO₄²⁻, H₂PO₄⁻, SiO₄⁴⁻, AlO₂⁻, SiO₃⁻ und /oder [AlO₂)₁₂(SiO₂)₂]²⁻, vorzugsweise Cl⁻ und Br⁻, besonders bevorzugt Cl⁻.

6. Das Kohlensäure-Addukt (KA) zur Verwendung gemäß Anspruch 3 und 5, wobei Schritt a) mindestens einen der folgenden Teilschritte umfasst:
a1) Kühlen des Lösungsmittels, vorzugsweise Wasser, auf 3 bis 8 °C, vorzugsweise 5 °C und/oder a2) Einbringen von CO₂ in das Lösungsmittel, vorzugsweise bis zu einer Sättigungskonzentration von 3 bis 10 g/l, mehr bevorzugt bis zu einer Sättigungskonzentration 4,5 bis 7,5 g/l, vorzugsweise beträgt der pH-Wert der Lösung nach der Sättigung mit CO₂ ≤ 3,0 bis 6,0, noch mehr bevorzugt ≤ 4,3 bis 4,8 und/oder
a3) Lagerung der Lösung (A) bei 1 bis 10 °C, vorzugsweise für mindestens 30 min, mehr bevorzugt für mindestens 50 min, noch mehr bevorzugt für mindestens 60 min; bis höchstens 5 Tage (120 h); vorzugsweise erfolgt die Lagerung bei 3 bis 8 °C vorzugsweise für mindestens 30 min, mehr bevorzugt für mindestens 50 min, noch mehr bevorzugt für mindestens 60 min; bis höchstens 5 Tage (120 h);
vorzugsweise umfasst Schritt a) alle Teilschritte a1), a2) und a3),
vorzugsweise werden die Teilschritte a1), a2) und a3) in der Reihenfolge a2) folgt auf a1) und a3) folgt auf a2) durchgeführt.

7. Das Kohlensäure-Addukt (KA) zur Verwendung gemäß einem der Ansprüche 3, 5 und 6, wobei
i) die Base (BA) in Schritt b) ein Hydrogencarbonat oder ein Carbonat, mehr bevorzugt ein Hydrogencarbonat, noch mehr bevorzugt Natriumhydrogencarbonat ist und/oder
ii) der Gehalt an CO₂ in der Lösung, die dem Schritt d) unterzogen wird, mindestens 6 g/l, vorzugsweise mindestens 10 g/l, mehr bevorzugt mindestens 12 g/l, noch mehr bevorzugt mindestens 14 g/l und ganz besonders bevorzugt mindestens 15 g/l beträgt und das Amin (AM) auch in Form eines Salzes eingesetzt werden kann.

8. Das Kohlensäure-Addukt (KA) zur Verwendung gemäß einem der Ansprüche 3 und 5 bis 7, wobei Schritt c) mindestens einen der folgenden Teilschritte umfasst:
c1) Auflösen des mindestens einen Amins (AM) in Lösung (A) oder (A1) unter Erhalt der Lösung (B) und/oder
c2) Zugabe von Lösung (A) zur Lösung (B) unter Erhalt der Lösung (B1) und/oder c3) Anreicherung der Lösung (B) oder (B1) mit CO₂ und/oder
c4) Lagerung der Lösung (B) oder (B1) bei 1 bis 10°C, vorzugsweise 3 bis 8 °C, für mindestens 1 h vorzugsweise 24 h bis 120 h, noch mehr bevorzugt 24 bis 72 h, und/oder
c5) Anreicherung der Lösung (B) oder (B1) mit CO₂ auf eine Konzentration von mindestens 6 g/l, vorzugsweise mindestens 10 g/l, mehr bevorzugt mindestens 12 g/l, noch mehr bevorzugt mindestens 14 g/l und ganz besonders bevorzugt mindestens 15 g/l;
wobei optional
i) die Konzentration des Amins (AM) in Lösung (B) oder bei Ausführung von Teilschritt c2) in Lösung (B1) 0,01 bis 0,25 g/ml, vorzugsweise 0,03 bis 0,20 g/ml, mehr bevorzugt 0,08 bis 0,15 g/ml beträgt und/oder
ii) der pH-Wert der Lösung (B) oder (B1) nach der Durchführung von Schritt c5) ≤ 7,0 beträgt und/oder
iii) das Verhältnis des Amins (AM) zur Base (BA), bei Durchführung des Schrittes b), in Lösung (B) 2 bis 5, mehr bevorzugt 3 bis 4, noch mehr bevorzugt 3,23 bis 3,26 [g/g] beträgt und/oder
iv) in Schritt c1) das mindestens eine Amin (AM), das mindestens eine Amin (AM) als Säureadditionssalz, vorzugsweise als Hydrohalogenid, Hydrogensulfat, Hydrogensulfit, Hydrogenphosphat, Hydromesylat, Hydrotosylat, Hydroacetat, Hydroformiat, Hydropropanoat, Hydromalonat, Hydrosuccinat, Hydrofumarat, Hydroxalat, Hydrotartrat, Hydrocitrat, Hydromaleat, mehr bevorzugt als Hydrochlorid oder Hydrobromid umfasst.
vorzugsweise umfasst Schritt c) alle Teilschritte c1), c2), c3), c4) und c5);
vorzugsweise werden die Teilschritte c1), c2), c3), c4) und c5) in der Reihenfolge c2) folgt auf c1), c3) folgt auf c2), c4) folgt auf c3), c5) folgt auf c4) durchgeführt.

9. Das Kohlensäure-Addukt (KA) zur Verwendung gemäß einem der Ansprüche 3 und 5 bis 8, wobei in Schritt d):
i) die Lösung (B) oder (B1) bei -100 °C bis -20 °C, vorzugsweise bei -90 °C bis -30 °C, noch mehr bevorzugt bei -80 bis -40 °C und ganz besonders bevorzugt bei -70 bis -50 °C eingefroren wird und/oder
ii) die Lösung (B) oder (B1) innerhalb von 0,3 bis 60 min, vorzugsweise innerhalb von 1 bis 30 min, mehr bevorzugt innerhalb von 1,1 bis 10 min, noch mehr bevorzugt innerhalb von 1,5 bis 5 min eingefroren wird und/oder
iii) das Gefäß in dem sich die Lösung (B) oder (B1) während des Einfriervorgangs befindet, vorzugsweise im Kühlmedium, mit 10 bis 1000 rpm, vorzugsweise mit 50 bis 600 rpm, mehr bevorzugt mit 100 bis 400 rpm und noch mehr bevorzugt mit 200 bis 300 rpm rotiert wird und/oder
iv) die Lösung (B) oder (B1) mit einer Kühlrate von 10 bis 100 K/min, vorzugsweise mit 20 bis 80 K/min, mehr bevorzugt mit 30 bis 70 K/min und besonders bevorzugt mit 40 bis 60 K/min eingefroren wird.

10. Das Kohlensäure-Addukt (KA) zur Verwendung gemäß einem der Ansprüche 3 und 5 bis 8, wobei in Schritt e):
i) die eingefrorene Lösung (B) oder (B1) für 1,5 bis 4 Tage, vorzugsweise für 2,5 bis 4 Tage gelagert wird und/oder
ii) die eingefrorene Lösung (B) oder (B1) vorzugsweise bei -50 bis 0 °C, mehr bevorzugt bei -30 bis -5 °C, noch mehr bevorzugt bei -25 bis -10 °C, besonders bevorzugt bei -20 bis -15 °C gelagert wird.

11. Das Kohlensäure-Addukt (KA) zur Verwendung gemäß einem der Ansprüche 3 und 5 bis 10, wobei das Verfahren einen weiteren Schritt f) umfasst, der nach Schritt e) durchgeführt wird,
f) Trocknung der in Schritt e) gelagerten Lösung unter Erhalt von getrocknetem Kohlensäure-Addukt (KA),
wobei in Schritt f) optional
i) das Wasser aus der Lösung (B) oder (B1) bis zu einem Restgehalt von < 0,8 Gew.-%, vorzugsweise < 0,1 Gew.-% bezogen auf das Gesamtgewicht des Trocknungsproduktes (C) entfernt wird und/oder
ii) nicht im Kohlensäure-Addukt (KA) gebundenes CO₂ aus der Lösung (B) oder (B1) bis zu einem Restgehalt von < 0,8 Gew.-%, vorzugsweise < 0,1 Gew.-% bezogen auf das Gesamtgewicht des Trocknungsproduktes (C) entfernt wird und/oder
iii) die Trocknung mittels Lyophilisation durchgeführt wird und/oder
iv) während des Trocknens der Druck 0,01 bis 30 mbar, vorzugsweise 0,02 bis 20 mbar, mehr bevorzugt 0,03 bis 10 mbar, noch mehr bevorzugt 0,03 bis 0,5 mbar und ganz besonders bevorzugt 0,05 bis 0,1 mbar beträgt und vorzugsweise während des gesamten Trocknungsvorganges beibehalten wird und/oder
v) der Druck während des Trocknens gemäß iv) innerhalb von 10 h, vorzugsweise innerhalb von 7 h, mehr bevorzugt innerhalb von 5 h und besonders bevorzugt innerhalb von 4 h ab Evakuierungsbeginn erreicht wird und/oder
vi) die Temperatur während der gesamten Trocknung in Schritt f) 0 bis 20 °C, vorzugsweise 4 bis 18 °C, mehr bevorzugt 8 bis 16 °C beträgt und/oder
vii) die Gesamttrocknungszeit 10 bis 60, vorzugsweise 30 bis 55 h, mehr bevorzugt 41 bis 52 h beträgt.

12. Das Amin (AM) gemäß Anspruch 1, sowie das Kohlensäure-Addukt (KA) zur Verwendung gemäß einem der Ansprüche 2, 3 und 5 bis 11 und die pharmazeutische Zusammensetzung (PZ) zur Verwendung gemäß Anspruch 4, in der Behandlung von Superinfektionen mit Influenzaviren und *Aspergillus spp.,* vorzugsweise *Aspergillus fumigatus.*

13. Das Amin (AM) gemäß Anspruch 1, sowie das Kohlensäure-Addukt (KA) zur Verwendung gemäß einem der Ansprüche 2, 3 und 5 bis 12 und die pharmazeutische Zusammensetzung (PZ) zur Verwendung gemäß Anspruch 4 und 12, wobei die Applikation oral, parenteral, nasal, inhalativ, oder kutan erfolgt.
